# EUROPÄISCHE PATENTSCHRIFT

(11) **EP 3 506 993 B1**
(45) Veröffentlichungstag und Bekanntmachung des Hinweises auf die Patenterteilung: **06.01.2021**
(21) Anmeldenummer: 17764773.2
(22) Anmeldetag: 29.08.2017
(51) Int. Cl.: C07C 265/14, B01D 53/34, C07C 265/00, C07C 263/20, C07C 263/10, B01D 53/70, B01D 53/46, B01D 53/00, B01D 53/04, B01D 53/14

(54) **VERFAHREN ZUR HERSTELLUNG VON ISOCYANATEN**
PROCESS FOR THE PREPARATION OF ISOCYANATES
PROCÉDÉ DE FABRICATION D'ISOCYANATES

(30) Priorität: 01.09.2016 EP 16186777
(43) Veröffentlichungstag der Anmeldung: 10.07.2019
(73) Patentinhaber: Covestro Intellectual Property GmbH & Co. KG, 51373 Leverkusen (DE)
(72) Erfinder: KNAUF, Thomas, 41542 Dormagen (DE); MANZEL, Dirk, 47447 Moers (DE); PLATHEN, Peter, 47829 Krefeld (DE); SPRIEWALD, Jürgen, 25337 Kölln-Reisiek (DE)
(74) Vertreter: Levpat
(86) Internationale Anmeldenummer: PCT/EP2017/071602
(87) Internationale Veröffentlichungsnummer: WO 2018/041799

(56) Entgegenhaltungen:
- EP-B2- 1 575 908
- WO-A1-2004/056758

## Beschreibung

Die vorliegende Erfindung betrifft ein Verfahren zur Herstellung von Isocyanaten, bei welchem für die Verbrennnung vorgesehene Abgasströme vor deren Zufuhr in die Abgasverbrennung durch eine Adsorptionsvorrichtung geleitet und auf diese Weise an Lösungsmittel abgereichert werden, wobei die Adsorptionsvorrichtung mindestens zwei parallel geschaltete Adsorptionseinrichtungen umfasst, die wechselweise
(i) mit dem mindestens einen Abgasstrom beaufschlagt und
(ii) mit Wasserdampf regeneriert werden,
wobei während der Verfahrensstufe (i) ein an Lösungsmittel abgereicherter Abgasstrom erhalten wird und während der Verfahrensstufe (ii) ein Strom enthaltend Wasser und Lösungsmittel erhalten wird, dessen Lösungsmittelanteil in das Verfahren zur Herstellung von Isocyanaten rezykliert wird.

In vielen industriellen Produktionsprozessen, insbesondere Produktionsprozessen für chemische Produkte, fallen vom eigentlichen Produkt des Produktionsprozesses verschiedene gasförmige Verfahrensprodukte an, welche vor ihrer Entlassung als Abgase in die Umgebung aufgearbeitet werden müssen, um wirtschaftliche Verluste und Belastungen für die Umwelt zu minimieren. Solche Aufarbeitungsschritte können vielfältige Prozesse umfassen. Als Beispiele für solche Reinigungsoperationen seien die Wäsche, Absorption (z. B. von NOₓ), Adsorption, , Kondensation und Filtration genannt. Es ist im Stand der Technik üblich, solche auf diese Weise vorgereinigten gasförmigen Verfahrensprodukte nach Abschluss aller zur Vorreinigung durchgeführten Reinigungsoperationen (gegebenenfalls auch schon vorher) zu vereinen und den so erhaltenen Abgasstrom einer gemeinsamen Abgasverbrennung zuzuführen, in welcher das Abgas so vollständig verbrannt wird, dass die Verbrennungsgase (erforderlichenfalls nach einer Nachreinigung wie bspw. einer Basenwäsche zur Bindung saurer Bestandteile) unter Einhaltung behördlicher Umweltauflagen in die Umgebung entlassen werden können.

Ein Beispiel für einen industriellen Produktionsprozess, in dem gasförmige Verfahrensprodukte anfallen, ist die Produktion von Isocyanaten durch Phosgenierung der korrespondierenden Amine. In der Phosgenierung fällt ein gasförmiger Verfahrensstrom umfassend das Koppelprodukt Chlorwasserstoff und nicht umgesetztes Phosgen an. Auch Reste des eingesetzten Lösungsmittels (sowie Inerte oder auch überschüssiges Kohlenmonoxid aus der Phosgenherstellung) sind regelmäßig hierin enthalten. In sich anschließenden Aufarbeitungsschritten können weitere gasförmige Verfahrensprodukte, die teilweise auch Lösungsmittel enhalten, anfallen. In diesem Zusammenhang spielt die Rückgewinnung von Lösungsmittel aus den verschiedenen gasförmigen Verfahrensprodukten, die im Prozess an unterschiedlichen Stellen anfallen, eine Rolle. Mit Prozessen zur Lösemittelrückgewinnung via Destillation in Isocyanat-Produktionsprozessen befassen sich z. B. EP 1 575 908 B2 und EP 1 575 906 B1. In keiner dieser Druckschriften wird eine Lösemittelrückgewinnung unmittelbar vor der Abgasverbrennung beschrieben.

WO 2004/056758 A1 beschreibt eine Nachreinigung von Chlorwasserstoff aus der Chlorwasserstoff/Phosgentrennung durch Adsorption von Verunreingungen (Reste von Phosgen und Chlorbenzol) an Aktivkohle.

Es wäre wünschenswert, ein verlässliches Verfahren zur Lösungsmittelrückgewinnung aus Abgasströmen von Isocyanat-Produktionsanlagen vor deren Einleitung in die Abgasverbrennung zur Verfügung zu haben.

Es wäre ferner wünschenswert, ein solches Verfahren auf einfache Weise veränderten Randbedingungen wie bspw. veränderten Kosten für Lösungsmittel, Energie oder Abgasverbrennung anpassen zu können.

Diesem Bedarf Rechnung tragend ist ein Gegenstand der vorliegenden Erfindung ein **Verfahren zur Herstellung eines Isocyanats (1)**, umfassend die Schritte:
A. Umsetzung des zu dem Isocyanat (1) korrespondierenden Amins (2) mit Phosgen (3) unter Einsatz eines Lösungsmittels (4), wobei ein das Isocyanat (1) und Lösungsmittel (4) enthaltender flüssiger Strom sowie mindestens ein lösungsmittelhaltiger Abgasstrom erhalten werden;
B. Aufarbeitung des Isocyanat und Lösungsmittel enthaltenden flüssigen Stroms zur Isolierung des Isocyanats, wobei weiteres Lösungsmittel (4) eingesetzt werden kann und wobei ebenfalls mindestens ein lösungsmittelhaltiger Abgasstrom anfällt;
wobei
a) der mindestens eine lösungsmittelhaltige Abgasstrom aus Schritt A und/oder aus Schritt B in eine Adsorptionsvorrichtung zur Adsorption von Lösungsmittel (3020) geführt wird, welche mindestens zwei parallel geschaltete Adsorptionseinrichtungen (3021, 3022) umfasst, die wechselweise
   (i) mit dem mindestens einen Abgasstrom beaufschlagt und
   (ii) mit Wasserdampf regeneriert werden,
   wobei während der Verfahrensstufe a) (i) ein an Lösungsmittel abgereicherter Abgasstrom (220) erhalten wird und während der Verfahrensstufe a) (ii) ein Strom enthaltend Wasser und Lösungsmittel (230) erhalten wird, wobei
b)
   (i) der an Lösungsmittel abgereichterte Abgasstrom (220) einer Abgasverbrennung (6000) zugeführt wird und
   (ii) der Strom enthaltend Wasser und Lösungmittel (230) an Wasser abgereichert und danach in Schritt A und/oder in Schritt B zurückgeführt wird.

Ein weiterer Gegenstand der vorliegenden Erfindung ist eine zur Durchführung des erfindungsgemäßen Verfahrens geeignete **Anlage (10000) zur Herstellung eines Isocyanats (1) durch Phosgenierung des korrespondierenden Amins (2).**

Es folgt zunächst eine Kurzzusammenfassung verschiedener möglicher **Ausführungsformen der Erfindung:**
In einer **ersten Ausführungsform** der Erfindung, die mit allen anderen Ausführungsformen kombiniert werden kann, umfasst Schritt A:
I) Umsetzung des Amins (2) mit Phosgen (3) und Trennung des erhaltenen Verfahrensprodukts in den das Isocyanat und Lösungsmittel enthaltenden flüssigen Strom (60) und einen gasförmigen, Phosgen, Chlorwasserstoff und Lösungsmittel enthaltenden Strom (70);
   ferner umfasst Schritt B:
II) Abreicherung von Phosgen und Chlorwasserstoff aus dem flüssigen Strom (60) aus Schritt I) durch Auftrennung dieses flüssigen Stroms (60) in einen flüssigen, Lösungsmittel und Isocyanat enthaltenden Strom (80) und einen gasförmigen, Phosgen und Chlorwasserstoff enthaltenden Strom (90) in einer Destillationsvorrichtung (2100);
wobei die gasförmigen Ströme (70) und (90), optional nach Durchlaufen weiterer Reinigungsschritte, als Bestandteil, gegebenenfalls als alleiniger Bestandteil, des mindestens einen lösungsmittelhaltigen Abgasstroms dem Schritt a) zugeführt werden.

In einer **zweiten Ausführungsform** der Erfindung, die eine besondere Ausgestaltung der ersten Ausführungsform ist, schließen sich an Schritt B.II) die Schritte B.III) und B.IV) an:
III) Abreicherung von Lösungsmittel aus dem flüssigen Strom (80) aus Schritt II) durch Trennung dieses flüssigen Stroms (80) in einen gasförmigen, Lösungsmittel enthaltenden Strom (110) und eine flüssigen, Isocyanat enthaltenden Strom (100) in einer Destillationsvorrichtung (2200);
IV) Abreicherung von Phosgen aus dem gasförmigen Strom (110) aus Schritt III) durch Trennung dieses gasförmigen Stroms (110), bevorzugt nach dessen Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
wobei der gasförmige Strom (130), optional nach Durchlaufen weiterer Reinigungsschritte, als Bestandteil des mindestens einen lösungsmittelhaltigen Abgasstroms dem Schritt a) zugeführt wird.

In einer **dritten Ausführungsform** der Erfindung, die eine besondere Ausgestaltung der zweiten Ausführungsform ist, schließt sich an Schritt B.IV) Folgendes an:
V) Gewinnung eines flüssigen Isocyanat-Stroms (140) aus dem flüssigen Strom (100), wobei ein Nebenkomponenten und gegebenenfalls Lösungsmittel enthaltender gasförmiger Strom (150) anfällt, in einer Destillationsvorrichtung (2400), optional umfassend die Abtrennung von polymeren Isocyanatfraktionen in einer vorgeschalteten Einrichtung zur Polymerabtrennung (2410) als Strom (141).

In einer **vierten Ausführungsform** der Erfindung, die eine besondere Ausgestaltung der ersten, zweiten und dritten Ausführungsform ist, umfasst Schritt B zusätzlich Folgendes:
VI) Reinigung der gasförmigen Ströme (70) und (90) und, sofern vorhanden, des gasförmigen Stroms (130), gegebenenfalls nach Kondensation, durch Absorption in Lösungsmittel (4) unter Erhalt eines flüssigen, Lösungsmittel und Phosgen enthaltenden Stroms (160) und eines gasförmigen, Chlorwasserstoff und Lösungsmittel enthaltenden Stroms (170) in einer Absorptionsvorrichtung (2500).

In einer **fünften Ausführungsform** der Erfindung, die eine besondere Ausgestaltung der vierten Ausführungsform ist, schließt sich an Schritt B.VI) Folgendes an:
VII) Reinigung des gasförmigen Stroms (170) in Wasser oder Salzsäure als Absorptionsmittel (180) in einer weiteren Absorptionsvorrichtung (2600), wobei ein Salzsäure enthaltender Strom (190) und, bevorzugt nach Durchlaufen eines Brüdenkondensators (2610), ein gasförmiger, Lösungsmittel und gegebenenfalls gasförmige Nebenkomponenten enthaltender Strom (200) erhalten werden.

In einer **sechsten Ausführungsform** der Erfindung, die eine besondere Ausgestaltung der fünften Ausführungsform ist, wird in Schritt B.VII) der Brüdenkondensator (2610) durchlaufen, und dieser Schritt B.VII) umfasst zusätzlich Folgendes:
Trennung des im Brüdenkondensator (2610) erhaltenen flüssigen Stroms (191) in eine wässrige Phase (192) und eine organische Phase (193);
Rezyklierung der wässrigen Phase (192) als Bestandteil des Absorptionsmittels (180) in die Absorptionsvorrichtung (2600) und/oder
Rezyklierung der organischen Phase (193), bevorzugt nach Trockung, in mindestens einen der Schritte A.I), B.III), B.IV und B.VI).

In einer **siebten Ausführungsform** der Erfindung, die eine besondere Ausgestaltung der fünften und sechsten Ausführungsform ist, schließt sich an Schritt B.VII) Folgendes an:
VIII) Reinigung des gasförmigen, Lösungsmittel und gegebenenfalls gasförmige Nebenkomponenten enthaltenden Stroms (200) in einer Vorrichtung zur Aufarbeitung von Abgasströmen (3000), wobei
VIII-1) der aus der Absorptionsvorrichtung (2600), bevorzugt nach Durchlaufen des Brüdenkondensators (2610), stammende gasförmige Strom (200) in eine Vorrichtung zur Phosgenzersetzung (3010) geleitet wird, in welcher Phosgen katalytisch, bevorzugt an Aktivkohle, unter Verwendung von Wasser (260) zersetzt wird, wobei ein gasförmiger, Lösungsmittel und gegebenenfalls gasförmige Nebenkomponenten enthaltender Strom (210) und ein salzsaurer flüssiger Strom (270) erhalten werden, wobei der gasförmige Strom (210) in die Adsorptionsvorrichtung (3020) zur Adsorption von Lösungsmittel in Schritt a) geführt wird.

In einer **achten Ausführungsform** der Erfindung, die mit allen anderen Ausführungsformen der Erfindung, insbesondere mit der siebten Ausführungsform, kombinierbar ist, wird das Phosgen (3) für die Durchführung von Schritt A durch Umsetzung von Kohlenstoffmonoxid (300) mit Chlor (310) hergestellt, wobei ein Kohlenstoffmonoxid und Phosgen enthaltender Abgasstrom (320) anfällt, der, optional nach Durchlaufen weiterer Reinigungsschritte, dem der Abgasverbrennung zuzuführenden mindestens einen lösungsmittelhaltigen Abgasstrom zugeführt wird, und zwar insbesondere derart, dass der Abgasstrom (320), optional nach Durchlaufen weiterer Reinigungsschritte, vor Durchführung von Schritt VIII.1) mit dem gasförmigen Strom (200) vereinigt wird.

In einer **neunten Ausführungsform** der Erfindung, die eine besondere Ausgestaltung der achten Ausführungsform ist, wird der Kohlenstoffmonoxid und Phosgen enthaltende Abgasstrom (320) aus der Umsetzung von Kohlenstoffmonoxid (300) mit Chlor (310) in Lösungsmittel (4) einer Temperatur im Bereich von 0,0 °C bis -20,0 °C in einer Absorptionskolonne (4010) unter Erhalt eines an Phosgen abgereicherten Abgasstroms (330) gereinigt, wobei dieser Abgasstrom (330) vor Durchführung von Schritt VIII.1) mit dem gasförmigen Strom (200) vereinigt wird, wobei in der Absorptionskolonne (4010) ein Lösungsmittel- und Phosgen-haltiger Strom (340) anfällt, der der Absorptionsvorrichtung (2500) zugeführt wird.

In einer **zehnten Ausführungsform** der Erfindung, die mit allen anderen Ausführungsformen der Erfindung, insbesondere mit der vierten, fünften, sechsten, siebten, achten oder neunten Ausführungsform, kombinierbar ist, wird die Abreicherung des Wasser und Lösungmittel enthaltenden Stroms in Schritt b) (ii) an Wasser derart durchgeführt, dass dieser Strom kondensiert und dann in eine wässrige Phase (250) und eine organische Phase (240) getrennt wird, wobei die organische Phase (240) optional weiter getrocknet werden kann, wobei die Rückführung des nach Abreicherung von Wasser erhaltenen Stroms in Schritt A und/oder in Schritt B derart durchgeführt wird, dass die organische Phase (240), gegebenenfalls nach weiterer Trocknung, in Schritt A und/oder in Schritt B rezykliert wird, und zwar insbesondere in mindestens einen der Schritte A I), B.III), B.IV) und B.VI) rezykliert wird.

In einer **elften Ausführungsform** der Erfindung, die eine besondere Ausgestaltung der zehnten Ausführungsform ist, wird die wässrige Phase (250) als Bestandteil des in Schritt B.VII) eingesetzten Absorptionsmittels eingesetzt.

In einer **zwölften Ausführungsform** der Erfindung, die eine besondere Ausgestaltung der zweiten bis elften Ausführungsform, insbesondere der dritten bis elften Ausführungsform, ist, werden die Einrichtungen (2200) und (2300), insbesondere die Einrichtungen (2200), (2300), (2400) und, sofern vorhanden, (2410) bei einem gegenüber Atmosphärendruck vermindertem Druck betrieben, und die Abgasströme der hierfür erforderlichen Vakuumerzeugungsanlagen werden ohne Abkühlung oder nach Abkühlung auf eine Temperatur nicht tiefer als 1,0 °C, optional nach Durchlaufen einer Vorrichtung zur katalytischen Phosgenzersetzung (3010), dem Schritt a) zugeführt.

In einer **dreizehnten Ausführungsform** der Erfindung, die mit allen anderen Ausführungsformen kombiniert werden kann, wird das Amin (2) ausgewählt aus der Gruppe bestehend aus Methylendiphenylendiamin, Polymethylenpolyphenylenpolyamin, einem Gemisch aus Methylendiphenylendiamin und Polymethylenpolyphenylenpolyamin, Toluylendiamin, Xylylendiamin, 1,5-Pentandiamin, Hexamethylendiamin, Isophorondiamin und Naphthyldiamin. Bevorzugt wird das Amin ausgewählt aus der Gruppe bestehend aus Methylendiphenylendiamin, Polymethylenpolyphenylenpolyamin, einem Gemisch aus Methylendiphenylendiamin und Polymethylenpolyphenylenpolyamin, Toluylendiamin, Xylylendiamin, Hexamethylendiamin, Isophorondiamin und Naphthyldiamin.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

Das erfindungsgemäße Verfahren ist grundsätzlich zur Herstellung beliebiger aromatischer, aliphatischer und araliphatischer *Isocyanate (1)* geeignet. Bevorzugt eingesetzt wird das erfindungsgemäße Verfahren zur Herstellung von Methylendiphenylendiisocynat (aus Methylendiphenylendiamin), Polymethylenpolyphenylenpolyisocyanat (aus Polymethylenpolyphenylenpolyamin), Gemischen aus Methylendiphenylendiisocynat und Polymethylenpolyphenylenpolyisocyanat, Toluylendiisocyanat (aus Toluylendiamin), Xylylendiisocyanat (aus Xylylendiamin), 1,5-Pentandiisocyanat (aus 1,5-Pentandiamin), Hexamethylendiisocyanat (aus Hexamethylendiamin), Isophorondiisocyanat (aus Isophorondiamin) und Naphthyldiisocyanat (aus Naphthyldiamin), besonders bevorzugt von Methylendiphenylendiisocyanat, Gemischen aus Methylendiphenylendiisocyanat und Polymethylenpolyphenylenpolyisocyanat sowie Toluylendiisocyanat. Ganz besonders bevorzugt eignet sich das erfindungsgemäße Verfahren zur Herstellung von Methylendiphenylendiisocyanat und Gemischen aus Methylendiphenylendiisocyanat und Polymethylenpolyphenylenpolyisocyanat. Methylendiphenylendiisocyanat wird auch als Diamin der Diphenylmethanreihe bezeichnet. Polymethylenpolyphenylenpolyisocyanat wird auch als Polyamin der Diphenylmethanreihe bezeichnet.

***Schritt A*** kann in der Gas- oder Flüssigphase erfolgen. Bei Flüssigphasenphosgenierungen ist es üblich, Amin (2) und Phosgen (3) in einem Lösugsmittel (4) zu lösen und zur Reaktion zu bringen. Bei Gasphasenphosgenierungen ist es üblich, das rohe gasförmige Verfahrensprodukt der Umsetzung von Amin und Phosgen durch Kontaktieren mit Lösungsmittel (4) (und gegebenenfalls weiterer Flüssigkeit wie rezykliertem Isocyanat) bei einer Temperatur unterhalb des Siedepunkts des Isocyanats (1) und oberhalb des Zersetzungspunktes des korrespondierenen Carbamoylchlorids rasch abzukühlen, wobei das gebildete Isocyanat in die sich ausbildende Flüssigphase übergeht. Unabhängig von der Verfahrensführung entsteht also ein *das Isocyanat (1) und Lösungsmittel (4) enthaltender flüssiger Strom.* Daneben entstehen immer auch gasförmige Verfahrensprodukte die weiter aufgearbeitet werden, wobei wertvolle Rohstoffe weitestmöglich zurückgewonnen werden. Nach Ausschöpfung der bisher im Stand der Technik möglichen und wirtschaftlich sinnvollen Möglichkeiten der Aufarbeitung der gasförmigen Verfahrensprodukte verbleiben stets *Abgasströme.* Da die vollständige Rückgewinnung von Lösungsmittel aus allen gasförmigen Verfahrensprodukten infolge des dazu erforderlichen unverhältnismäßig hohen Energieeinsatzes unwirtschaftlich wäre, fällt in einem Verfahren zur Herstellung von Isocyanaten regelmäßig *mindestens ein **lösungsmittelhaltiger** Abgasstrom* an. Dieser mindestens eine lösungmittelhaltige Abgasstrom wird erfindungsgemäß vor der *Abgasverbrennung (Schritt b) (i))* noch durch einen *Adsorptionsprozess (Schritt a))* weiter an Lösungsmittel abgereichert. Dabei ist es bevorzugt, dass diese Lösungsmittelabreicherung unmittelbar vor der Abgasverbrennung stattfindet, d. h. zwischen Schritt a) und Schritt b) (i) finden bevorzugt keine weiteren Verfahrensschritte statt.

Geeignete erfindungsgemäß einsetzbare inerte *Lösungsmittel (4)* sind dabei unter den Reaktionsbedingungen inerte Lösungsmittel wie z. B. Monochlorbenzol, Dichlorbenzol (insbesondere das *ortho-Isomer),* Dioxan, Toluol, Xylol, Methylenchlorid, Perchlorethylen, Trichlorfluormethan oder Butylacetat. Das inerte Lösungsmittel (4) ist bevorzugt im Wesentlichen frei von Isocyanat (Massenanteil < 100 ppm angestrebt) und im Wesentlichen frei von Phosgen (Massenanteil < 100 ppm angestrebt), worauf bei Einsatz von Recycleströmen zu achten ist. Bevorzugt wird daher nach einem Verfahren, wie in EP 1 854 783 A2 beschrieben, gearbeitet. Die Lösungsmittel können einzeln oder als beliebige Gemische der beispielhaft genannten Lösungsmittel eingesetzt werden. Vorzugsweise werden Monochlorbenzol (MCB) oder *ortho*-Dichlorbenzol (ODB) eingesetzt.

***Schritt B*** kann im Rahmen der vorliegenden Erfindung wie im Stand der Technik üblich durchgeführt werden. Bevorzugte Ausführungsformen werden weiter unten geschildert.

***Schritt a)*** des erfindungsgemäßen Verfahrens umfasst die Abreicherung des zu verbrennenden mindestens einen lösungsmittelhaltigen Abgasstroms an Lösungsmittel in einer *Adsorptionsvorrichtung zur Adsorption von Lösungsmittel. Diese umfasst mindestens zwei parallel geschaltete Adsorptionseinrichtungen,* enthaltend ein Adsorptionsmittel (bevorzugt Aktivkohle). Eine dieser Adsorptionseinrichtungen wird in a) (i) mit dem anschließend zu verbrennenden mindestens einen lösungsmittelhaltigen Abgasstrom beaufschlagt, und zwar längstens bis zur Sättigungsgrenze des Adsorptionsmittels. Dann wird der zu verbrennende mindestens eine lösungsmittelhaltige Abgasstrom in a) (ii) in die zweite Adsorptionseinrichtung enthaltend frisches oder regeneriertes Adsorptionsmittel geleitet, und zeitgleich wird das in der ersten Adsorptionseinrichtung enthaltende Adsorptionsmittel mit Wasserdampf regeneriert. Bevorgzugte Ausgestaltungen des Schritts a) werden weiter unten näher erläutert.

In ***Schritt b) (i)** wird der an Lösungsmittel abgereicherte Abgasstrom (220) bevorzugt unmittelbar (d. h. bevorzugt ohne Zwischenschritte zwischen Schritt a) (i) und b (i)) einer Abgasverbrennung* zugeführt. Die Abgasverbrennung ist ein dem Fachmann bekannter Schritt in der chemischen Verfahrenstechnik und kann im Rahmen der vorliegenden Erfindung wie im Stand der Technik üblich durchgeführt werden.

Die *Abreicherung des in Schritt a) ii) erhaltenen Wasser und Lösungmittel enthaltenden Stroms in **Schritt b) (ii)** an Wasser* wird zweckmäßigerweise derart durchgeführt, dass dieser Strom kondensiert und dann in eine wässrige Phase und eine organische Phase getrennt wird, wobei die organische Phase optional weiter getrocknet werden kann, und bei welchem die Rückführung des nach Abreicherung von Wasser erhaltenen Stroms in Schritt A und/oder in Schritt B derart durchgeführt wird, dass die organische Phase, gegebenenfalls nach weiterer Trocknung, als Bestandteil des Lösungsmittels (4) in Schritt A und/oder in Schritt B eingesetzt wird. Bevorgzugte Ausgestaltungen dieses Schritts werden weiter unten näher erläutert.

Es folgt eine Detailbeschreibung unter Verweis auf die beigefügten Zeichnungen, wobei wiederum unterschiedliche Ausführungsformen beliebig miteinander kombinierbar sind, sofern sich aus dem Zusammenhang für den Fachmann nicht das Gegenteil ergibt.

Die kontinuierliche oder semi-kontinuierliche, bevorzugt kontinuierliche, **Produktion des Isocyanats in Schritt A** erfolgt in einem aus dem Stand der Technik bekannten Verfahren. Geeignete Verfahren sind beispielsweise in EP 2 077 150 A1, EP 1 616 857 A1, EP 1 873 142 A1, EP 0 716 079 A1 und EP 0 314 985 B1 (Flüssigphasenverfahren) sowie EP 2 196 455 A1, EP 1 449 826 A1 und WO 2015/144681 A1 (Gasphasenverfahren) beschrieben. Konzentrationen und Mengenströme der Edukte Amin (2) und Phosgen (3) werden dabei jedoch bevorzugt so gewählt, dass sich in der Vermischung der Reaktionspartner ein molares Verhältnis von Phosgen zu primären Aminogruppen von 1,1 : 1 bis 30 : 1, besonders bevorzugt von 1,25 : 1 bis 3 : 1, einstellt.

In einer bevorzugten Ausführungsform des erfindungsgemäßen Verfahrens umfasst Schritt A:
I) Umsetzung des Amins (2) mit Phosgen (3) und Trennung des erhaltenen Verfahrensprodukts in den das Isocyanat und Lösungsmittel enthaltenden flüssigen Strom (60) und einen gasförmigen, Phosgen, Chlorwasserstoff und Lösungsmittel enthaltenden Strom (70).

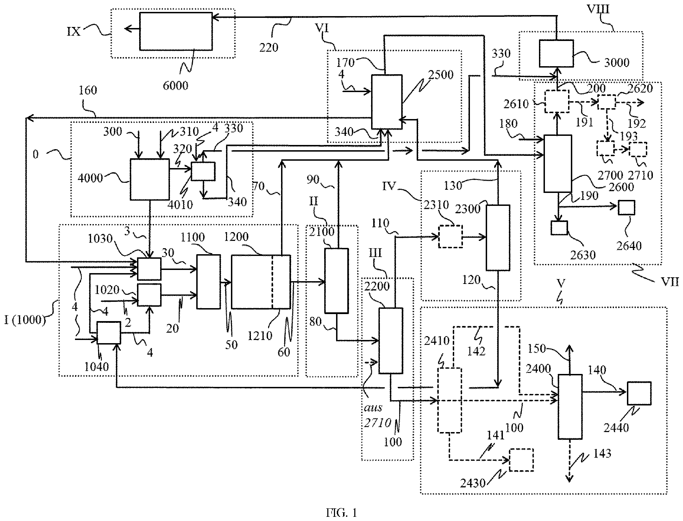

Amin (2) und Phosgen (3) werden aus entsprechenden Vorratsbehältern (1020, 1030) der Vermischung in (1100) zugeführt (siehe **FIG. 1****).** Dies geschieht bevorzugt in Form von Lösungen (20, 30) im Lösungsmittel (4). Bei Gasphasenprozessen kann auf die Lösung der Reaktanden in Lösungsmittel (4) verzichtet werden. Geeignete Mischeinrichtungen 1100 sind aus dem Stand der Technik hinreichend bekannt. Nach der Vermischung wird das Reaktionsgemisch (50) in den Reaktionsraum (1200) geleitet. Dieser ist eine Verweilzeiteinrichtung, in welcher das in der Mischeinrichtung 1100 erhaltene Gemisch ausreichende Gelegenheit zum Ausreagieren erhält. Geeignete Apparate sind aus dem Stand der Technik hinlänglich bekannt. Die Trennung des rohen Verfahrensproduktes in den flüssigen Strom 60 und den gasförmigen Strom 70 erfolgt im Reaktionsraum selber oder in einer nachgeschalteten Abscheidereinrichtung 1210. Es ist auch möglich, die Mischeinrichtung und den Reaktionsraum oder die Mischeinrichtung, den Reaktionsraum und die Abscheidereinrichtung oder den Reaktionsraum und die Abscheidereinrichtung in einen einzigen Apparat (z. B. in einen entsprechenden Reaktor) zu integrieren. Erfindungsgemäß können auch mehrere Mischeinrichtungen und/oder Reaktionsräume und/oder, sofern vorhanden, Abscheidervorrichtungen, seriell oder parallel geschaltet sein; z. B. in Form einer Kaskade mehrerer in Serie geschalteter Reaktoren. Das in 1200 erhaltene Verfahrensprodukt trennt sich in eine Flüssigphase (60), enthaltend neben dem gewünschten Isoyanat gelösten Chlorwasserstoff, überschüssiges gelöstes Phosgen und Lösungsmittel, und eine Gasphase (70), enthaltend Chlorwasserstoffgas, überschüssiges gasförmiges Phosgen und gasförmiges Lösungsmittel. Flüssig- und Gasphase werden dem Reaktionsraum (1200) oder der Abscheideeinrichtung (1210) getrennt entnommen. An den Reaktionsraum kann sich erforderlichenfalls eine Vorrichtung zur Spaltung von Carbaminsäurechlorid(nicht gezeigt in FIG. 1) anschließen. Die Flüssigphase (60) durchläuft in einem solchen Fall diese Vorrichtung, bevor sie der Aufarbeitung in Schritt B unterworfen wird. Die dabei entstehende an Chlorwasserstoff angereicherte Gasphase wird bevorzugt mit der Gasphase (70) vereint und gemeinsam weiter aufgearbeitet.

Diese **Aufarbeitung in Schritt B** umfasst in dieser Ausführungsform mindestens den Schritt II):
II) Abreicherung von Phosgen und Chlorwasserstoff aus dem flüssigen Strom (60) aus Schritt I) durch Auftrennung dieses flüssigen Stroms (60) in einen flüssigen, Lösungsmittel und Isocyanat enthaltenden Strom (80) und einen gasförmigen, Phosgen und Chlorwasserstoff enthaltenden Strom (90) in einer Destillationsvorrichtung (2100; sog. "Entphosgenierkolonne").

Die weitere Abtrennung von Chlorwasserstoff und Phosgen aus dem flüssigen Roh-Isocyanat-Strom 60 in der sog. Entphosgenierkolonne 2100 in B.II) kann nach einem beliebigen aus dem Stand der Technik bekannten Verfahren erfolgen, bevorzugt wie in EP 1 854 783 B1, insbesondere in den Abschnitten [0018] und [0023] beschrieben.

In den beschriebenen Schritt I) und II) fallen die gasförmigen Ströme (70) und (90) an. Diese werden, bevorzugt nach Durchlaufen weiterer Reinigungsschritte, als Bestandteil, gegebenenfalls als alleiniger Bestandteil, des mindestens einen lösungsmittelhaltigen Abgasstroms dem Schritt a) zugeführt. In solchen weiteren Reinigungsschritten wird die Zusammensetzung der Ströme (70) und (90) verändert (z. B. können diese Ströme an Phosgen, Chlorwasserstoff und/oder Lösungsmittel abgereichert werden).

Neben den erwähnten gasförmigen Strömen (70) und (90) können noch an weiteren Stellen des Verfahrens gasförmige Ströme anfallen, die auf die geschilderte Weise Bestandteil der Aufarbeitung der gasförmigen Verfahrensprodukte des erfindungsgmäßen Verfahrens zur Herstellung eines Isocyanats werden. Ein Beispiel dafür sind gasförmige Ströme, die in der **weiteren Abtrennung von Lösungsmittel aus dem Strom (80)** über den Strom (130) resultieren. Bevorzugt geschieht dies in der Weise, dass sich an Schritt B.II) anschließt:
III) Abreicherung von Lösungsmittel aus dem flüssigen Strom (80) aus Schritt II) durch Trennung dieses flüssigen Stroms (80) in einen gasförmigen, Lösungsmittel enthaltenden Strom (110) und eine flüssigen, Isocyanat enthaltenden Strom (100) in einer Destillationsvorrichtung (2200; sog. "Lösungsmittelkolonne");
IV) Abreicherung von Phosgen aus dem gasförmigen Strom (110) aus Schritt III) durch Trennung dieses gasförmigen Stroms (110), bevorzugt nach dessen Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300; sog. "Lösungsmittelstripper").

Schritt B.III) kann nach einem beliebigen aus dem Stand der Technik bekannten Verfahren erfolgen, bevorzugt wie in EP 1 854 783 B1, insbesondere in den Abschnitten [0024] bis [0027], beschrieben. Die Destillationsvorrichtung (2200) kann auch zwei oder mehr in Serie geschaltete Destillationskolonnen umfassen (in FIG. 1 wird aus Gründen der zeichnerischen Vereinfachung diese Möglichkeit nicht gezeigt).

Schritt B.IV) kann nach einem beliebigen aus dem Stand der Technik bekannten Verfahren erfolgen, bevorzugt wie in EP 1 854 783 B1, insbesondere in den Abschnitten [0027] und [0028], beschrieben.

In Schritt B.IV) fällt der gasförmige Strom (130) an. Dieser wird, bevorzugt nach Durchlaufen weiterer Reinigungsschritte, als Bestandteil des mindestens einen lösungsmittelhaltigen Abgasstrom dem Schritt a) zugeführt. Diesbezüglich kann auf die Ausführungen zu den gasförmigen Strömen (70) und (90) verwiesen werden.

Die erwähnten **weiteren Reinigungsschritte der Gasströme** (70), (90) bzw. (130) vor der Durchführung von Schritt a) können vielfältig ausgestaltet sein. In einer bevorzugten Ausführungsform des erfindungsgemäßen Verfahrens umfasst Schritt B zusätzlich den Schritt VI):
VI) Reinigung (d. h. weitgehende Befreiung von Phosgen) der gasförmigen Ströme (70) und (90) und, sofern vorhanden, des gasförmigen Stroms (130), durch Absorption in Lösungsmittel (4) unter Erhalt eines flüssigen, Lösungsmittel und Phosgen enthaltenden Stroms (160) und eines gasförmigen, Chlorwasserstoff und Lösungsmittel enthaltenden Stroms (170) in einer Absorptionsvorrichtung (2500; sog. "Phosgenabsorber"), wobei bevorzugt die gasförmigen Ströme (70) und (90) zunächst vereint werden und der vereinigte Strom aus (70) und (90) sowie, sofern vorhanden, der Strom (130), jeweils kondensiert und dann flüssig in die Absorptionsvorrichtung (2500) eingetragen werden.

Schritt VI) kann nach einem beliebigen aus dem Stand der Technik bekannten Verfahren erfolgen, bevorzugt wie in EP 2 093 215 A1 beschrieben.

Der in Schritt VI) gebildete Strom (170) kann grundsätzlich Schritt a) zugeführt werden. Es ist jedoch bevorzugt, den gasförmigen Strom (170) vorher weiter aufzureinigen. Hiervon umfasst ist eine **Abreicherung des Chlorwasserstoffgehalts** in einem Schritt VII):
VII) Reinigung des gasförmigen Stroms (170) in Wasser oder Salzsäure als Absorptionsmittel (180) in einer weiteren Absorptionsvorrichtung (2600; "HCl-Absorptionskolonne"), wobei ein Salzsäure enthaltender Strom - gegebenenfalls mit Spuren an Lösungsmittel - (190) und, bevorzugt nach Durchlaufen eines Brüdenkondensators (2610), ein gasförmiger, Lösungsmittel und gegebenenfalls gasförmige Nebenkomponenten enthaltender Strom (200) erhalten werden.

Schritt VII) kann nach einem beliebigen aus dem Stand der Technik bekannten Verfahren erfolgen. Bevorzugt sind Verfahrensweisen, wie in EP 2 021 275 B1 beschrieben.

Als Absorptionsmittel (180) wird Wasser (z. B. Dampfkondensat) oder Salzsäure einer Konzentration im Bereich von 0,50 Massen-% bis 15,0 Massen-% (verdünnte Salzsäure) eingesetzt. Der Salzsäure enthaltende Strom (190) wird in den Salzsäuretank (2630) abgelassen. Neben dem Salzsäuretank (2630) ist ein weiter Lagertank (2640) so angeordnet, dass er bei Bedarf Strom (190) aufnehmen kann. Dieser sog. "Dünnsäuretank" (2640) wird dann benutzt, wenn die Zusammensetzung des Stroms (190) von der für Salzsäure geforderten Spezifikation signifikant abweicht.

Durch die bei der Absorption des Chlorwasserstoffs freiwerdende Wärme wird in Strom (170) enthaltendes Lösungsmittel überwiegend bis vollständig in den Gasstrom (200) überführt.

In der bevorzugten Ausführungsform unter Einsatz des Brüdenkondensators (2610) wird in demselben ein flüssiger Strom (191) erhalten. Der Strom (191) enthält im Allgemeinen wässrige und organische Bestandteile. In dieser Ausführungsform kann es daher zweckmäßig sein, den Strom (191) in einer Phasentrennvorrichtung (2620) in eine wässrige Phase (192) und eine organische Phase (193) zu trennen. Die wässrige Phase (192) wird bevorzugt - insbesondere bevorzugt nach Ablassen in den Dünnsäuretank (2640) - als Bestandteil des Absorptionsmittels (180) in die HCl-Absorptionskolonne (2600) zurückgeführt. Die wassergesättigte organische Phase (193) wird zur weiteren Verwendung in einem Vorlagebehälter (2700) gesammelt und bevorzugt nach Trockung (insbesondere über Molsiebe in einem Trocknungsbehälter 2710) in mindestens einen der Schritte A.I) und B.III) rezykliert. Dies kann geschehen, indem die organische Phase (193) nach Trocknung frischem Lösungsmittel (4), das in A.I) eingesetzt wird, zugemischt oder direkt in den Lösungsmitteltank (1040) geleitet wird. Es ist auch möglich, die organische Phase (193), bevorzugt nach Trocknung, in die Destillationskolonne (2200) einzuführen, bspw. indem sie mit dem Strom (80) vermischt oder wie in FIG. 1 gezeigt unabhängig von Strom (80) in diese Destillationskolonne eingeführt wird, und auf diese Weise über die Ströme (110) [aus der Destillationsvorrichtung (2200), B.III)] und (120) [(aus der Destillationsvorrichtung (2300), B.IV)] in den Lösungsmitteltank (1040) zu führen. Es ist ferner möglich, die organische Phase (193) nach Trocknung als Bestandteil des in der Absorptionsvorrichtung (2500) eingesetzten Lösungsmittels (4) einzusetzen (B-VI).

Der Absorptionsprozess in Schritt VII) kann auch mit einer direkten oder indirekten Strippung verbunden werden, um sicherzustellen, dass der Lösungsmittelanteil in Strom (190) möglichst gering ist.

Der - insbesondere bei zusätzlicher Strippung - bevorzugte Einsatz des Brüdenkondensators (2610), der mit Kühlwasser mit Temperaturen im Bereich von 0,5 °C bis 60,0 °C, bevorzugt 10,0 °C bis 30,0 °C, betrieben wird, bietet eine einfache Möglichkeit, den gasförmigen Kopfstrom der Kolonne (2600) so weit wie mit vertretbarem Aufwand möglich zu kondensieren. Durch geeignete Wahl der Kondensationstemperatur kann zudem mit beeinflusst werden, zu welchen Anteilen Lösungsmittel, an dieser Stelle des Verfahrens auskondensiert oder in Schritt a) zurückgewonnen wird.

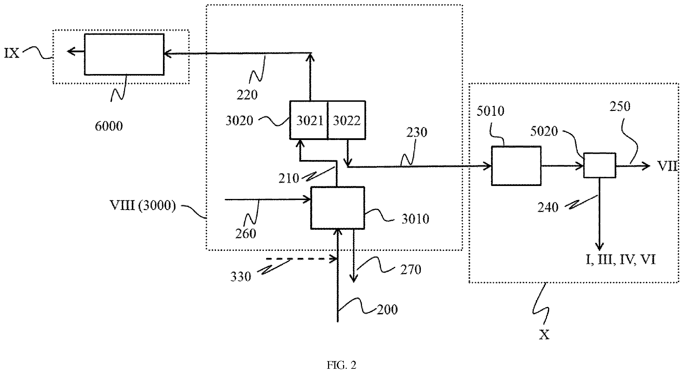

Der gasförmige Strom (200) kann grundsätzlich dem Schritt a) zugeführt werden. Es ist jedoch bevorzugt, diesen Strom vorher noch weiter zu reinigen und insbesondere bisher **nicht abgetrennte Reste an Phosgen zu zersetzen.** Zu diesem Zweck schließt sich in einer bevorzugten Ausführungsform an Schritt B.VII) an:
VIII) Reinigung des gasförmigen, Lösungsmittel und gegebenenfalls gasförmige Nebenkomponenten enthaltenden Stroms (200) in einer Vorrichtung zur Aufarbeitung von Abgasströmen (3000),
wobei (siehe **FIG. 2** für Details)
VIII-1) der aus der Adsorptionsvorrichtung (2600), bevorzugt nach Durchlaufen des Brüdenkondensator (2610), stammende gasförmige Strom (200) in eine Vorrichtung zur Phosgenzersetzung (3010; "Phosgenvernichtung") geleitet wird, in welcher Phosgen katalytisch, bevorzugt an Aktivkohle, unter Verwendung von Wasser (260) zersetzt wird, wobei ein gasförmiger, Lösungsmittel und gegebenenfalls gasförmige Nebenkomponenten enthaltender Strom (210) und ein salzsaurer flüssiger Strom (270) erhalten werden, wobei der gasförmige Strom (210) in die Adsorptionsvorrichtung (3020) zur Adsorption von Lösungsmittel in Schritt a) geführt wird.

Die Vorrichtung zur Zersetzung von Phosgen (3010) wird dabei bevorzugt so betrieben, dass Phosgen katalytisch, bevorzugt an Aktivkohle, unter Verwendung von Wasser einer Temperatur im Bereich von 5,0 °C bis 50,0 °C, bevorzugt 20,0 °C bis 40,0 °C zersetzt wird. Das kalte Wasser kann Betriebswasser, Dampf-Kondensat, vollentsalztes Wasser (VE-Wasser) oder eine Mischung mindestens zweier davon sein. Der Einsatz von Dampf-Kondensat ist bevorzugt. Die Phosgenzersetzung erfolgt bevorzugt bei einem Druck im Bereich von 800 mbar bis 1000 mbar (absolut), bevorzugt 850 mbar bis 950 mbar (absolut), insbesondere in zwei in Reihe hintereinander geschalteten Rohrreaktoren (sog. "Phosgenvernichtungstürmen").

In dieser Ausführungsform der Erfindung umfasst der der Adsorptionsvorrichtung (3020) in Schritt a) zuzuführende Strom also den in der Phosgenzersetzung (3010) erhaltenen praktisch phosgenfreien gasförmigen, Lösungsmittel und gegebenenfalls (von Phosgen verschiedene) gasförmige Nebenkomponenten enthaltenden Strom (210). Der Schritt a) zuzuführende Strom kann auch ausschließlich aus diesem gasförmigen Strom (210) bestehen.

Die Vorrichtung zur Adsorption von Lösungsmittel (3020) wird bevorzugt so betrieben, dass der mindestens eine lösungsmittelhaltige Abgasstrom, insbesondere also Strom (210), bei einer Temperatur im Bereich von 20,0 °C bis 60,0 °C, bevorzugt 30,0 °C bis 45,0 °C, und einem Druck im Bereich von 850 mbar bis 1000 mbar (absolut), bevorzugt 920 mbar bis 980 mbar (absolut), über Aktivkohle geleitet wird, wobei das Lösungsmittel in der Adsorptionseinrichtung (3021) an der Aktivkohle so lange angelagert (*adsorbiert*) wird, dass die Sättigungsgrenze der Aktivkohle nicht überschritten wird. Im Gegensatz zur Phosgenzersetzung in (3010) wird in (3020) kein Wasser zugegeben. Nach Beendigung eines solchen Beladungsintervalls wird die Aktivkohle der Einrichtung (3021) sodann regeneriert, indem das auf der Aktivkohle adsorbierte Lösungsmittel durcht direkten Wasserdampfeintrag wieder von der Aktivkohle desorbiert wird (Verfahrensstufe a) (ii)). Das dabei entstehende heiße, wässrige Lösungsmittelgemisch (230) wird in einem Kondensator (5010) abgekühlt und in einer Phasentrennvorrichtung (5020) in eine wässrige Phase (250), die in den "Dünnsäuretank" (2640) geleitet wird, und in eine organische Phase (240), enthaltend das Lösungsmittel, die in den Lagerbehälter (2700) geleitet wird, separiert. Die zeitliche Länge eines Beladungsintervalls hängt von der Lösungsmittelkonzentration in dem mindesten einen lösungsmittelhaltigen Abgasstrom, also insbesondere Strom (210), und dem zur Verfügung stehenden Volumen an Aktivkohle ab. Der der Abgasverbrennung (6000) zuzuführende an Lösungsmittel abgereicherte Abgasstrom (220) wird intervallweise oder kontinuierlich, bevorzugt kontinuierlich, mit einem Analysator auf organischen Kohlenstoff überwacht. Die Dauer eines Beladungsintervalls wird so gewählt, dass es keine unerwünschten Lösungsmittelverluste über die Verbrennung gibt. Um eine kontinuierliche Abgasreinigung betreiben zu können, wird während der Regeneration der Aktivkohle der Adsorptionseinrichtung (3021) der mindestens eine lösungsmittelhaltige Abgasstrom, insbesondere also Abgasstrom (210), über einen zweite identische Adsorptionseinrichtung (3021) geführt und dort an Lösungsmittel abgereichert. Ein Beladungsintervall ist bevorzugt 1,0 Stunde bis 24 Stunden, besonders bevorzugt 1,5 Stunden bis 12 Stunden und ganz besonders bevorzugt 2,0 bis 6,0 Stunden lang.

Der in Schritt a) (i) erhaltene, an Lösungsmittel abgereicherte Abgasstrom (220) aus der Absorptionsvorrichtung (3020) wird der **Verbrennung (6000)** zugeführt. Der in Schritt a) (ii) erhaltene, Wasser und Lösungsmittel enthaltende Strom (230) wird (siehe FIG. 2)
X) bevorzugt derart an Wasser abgereichert, dass dieser Strom (230) in einem Kondensator (5010) kondensiert und dann in einer Phasentrennvorrichtung (5020) in eine wässrige Phase (250) und eine organische Phase (240) getrennt wird, wobei die organische Phase (240) optional weiter getrocknet werden kann. Die, gegebenenfalls weiter getrocknete, organische Phase wird dann in mindestens einen der Schritte A.I), B.III), B.IV) und B.VI) rezykliert. Die wässrige Phase (250) kann vorteilhafterweise als Bestandteil des in Schritt VII) eingesetzten Absorptionsmittels eingesetzt werden; zur Zwischenlagerung wird die wässrige Phase (250) bevorzugt in den "Dünnsäuretank" (2640) geleitet.

Die bevorzugte Trocknung der organischen Phase (240) kann beispielsweise durch Trocknung an Zeolithen erfolgen. Geeignete Zeolithe sind z. B Molsiebe mit einer Porenweite von 2,0 Ǻ bis 10 Ǻ, bevorzugt 3,0 Ǻ bis 5,0 Ǻ.

Die organische Phase (240) kann nach Trocknung frischem Lösungsmittel (4), das in A.I) eingesetzt wird, zugemischt oder direkt in den Lösungsmitteltank (1040) geleitet werden. Die, gegebenenfalls getrocknete, organische Phase (240) kann ebenfalls in die Destillationskolonne (2200) eingeführt werden, bspw. indem sie mit dem Strom (80) vermischt oder unabhängig von Strom (80) in diese Destillationskolonne eingeführt wird, und auf diese Weise über die Ströme (110) [aus der Destillationsvorrichtung (2200), B.III)] und (120) [(aus der Destillationsvorrichtung (2300), B.IV)] in den Lösungsmitteltank (1040) geführt werden. Es ist auch möglich, gegebenenfalls getrocknete, organische Phase (240) als Bestandteil des in der Absorptionsvorrichtung (2500) als Absorptionsmittel eingesetzten Lösungsmittels (4) zu verwenden.

Aus Sicherheitsgründen ist es in allen Ausführungsformen der Erfindung bevorzugt, das für die Durchführung von Schritt A erforderliche **Phosgen (3) vor Ort** durch Umsetzung von Kohlenstoffmonoxid (300) mit Chlor (310) in einer entsprechenden Vorrichtung (4000) **herzustellen.** Für die Ausführung dieses Schritts kann ein "Kaltvereiniger" entsprechend EP 1 640 341 B1 oder ein "Heißvereiniger" entsprechend EP 0 134 506 B1 benutzt werden. Bei der sogenannten - bevorzugt eingesetzten - Heißvereinigung (siehe EP 0 134 506 B1) wird Phosgen durch Umsetzung von Chlor mit Kohlenmonoxid in Aktivkohle als Katalysator enthaltenen Rohrreaktoren unter gleichzeitiger Nutzung der anfallenden Reaktionswärme zur Erzeugung von Dampf umgesetzt. Dabei wird in einem ersten, gekörnte Aktivkohle enthaltenen Rohrreaktor mit einem lichten Rohrdurchmesser von maximal 100 mm 95 Vol.-% bis 98 Vol.-% des eingesetzten Chlors mit überschüssigem Kohlenmonoxid bei Reaktionstemperaturen von über 250,0 °C zu Phosgen umgesetzt. Die hierbei anfallende Reaktionswärme wird durch Verdampfungskühlung einer bei 150,0 °C bis 320,0 °C siedenden Flüssigkeit oder mit einer nicht-siedenden Flüssigkeit abgeführt, deren Temperatur am Reaktoraustritt mittels Zwangsumlaufpumpen und Temperatursteuerung bei 150,0 °C bis 320,0 °C gehalten wird. Der den Reaktor verlassende flüssige oder dampfförmige Wärmeträger wird in einem mit Wasser als Kühlmedium beschickten Wärmetauscher unter Erzeugung von Dampf kondensiert und / oder auf eine unter der Temperatur des Wärmeträgers am Reaktoraustritt liegende Temperatur abgekühlt und in den Reaktor zurückgeführt. Die den Reaktor verlassenden Reaktionsgase werden auf eine Temperatur von 50,0 °C bis 120,0 °C abgekühlt und anschließend in einen gekörnte Aktivkohle enthaltenden zweiten Reaktor geleitet, dessen Temperatur auf 50,0 °C bis 100,0 °C thermostatisch eingestellt und in dem die Umsetzung zu Ende geführt wird, so dass das den zweiten Reaktor verlassende Phosgen einen Restchlorgehalt von weniger als 50 ppmv aufweist. Das am Kopf des Reaktors austretende Phosgen wird, wie oben beschrieben, kondensiert. In jedem Verfahren entsteht ein Kohlenstoffmonoxid und Reste an Phosgen enthaltender Abgasstrom (320), der, bevorzugt nach Durchlaufen weiterer Reinigungsschritte, dem der Abgasverbrennung (6000) zuzuführenden mindestens einen lösungsmittelhaltigen Abgasstrom aus Schritt a) zugeführt wird. Geeignete Reinigungsschritte umfassen dabei eine Absorption des Abgasstroms (320) in Lösungsmittel (4) einer Temperatur im Bereich von 0,0 °C bis -20,0 °C in einer Absorptionskolonne (sog. Phosgenwäscher; (4010)), wobei ein Teil des Phosgens aus dem Kohlenstoffmonoxid und Reste an Phosgen enthaltenden Abgasstrom (320) herausgewaschen werden. Der in der Absorptionskolonne (4010) anfallende Lösungsmittel- und Phosgen-haltige Strom (340) wird bevorzugt der Absorptionsvorrichtung (2500) zugeführt. Der an Phosgen abgereicherte restliche Abgasstrom (330), der noch Lösungsmittelspuren enthält, wird bevorzugt, wie in FIG. 1 gezeigt, der Phosgenzersetzung (3010) zugeführt. Auf diese Weise wird der Abgasstrom aus der Phosgenherstellung ebenfalls der erfindungsgemäßen Adsorption zugeführt.

Zur **Reindarstellung des gewünschten Isocyanats (1)** ist es bevorzugt, den nach Abtrennung des Lösungsmittels in der oben beschriebenen Lösungsmittelkolonne (2200) erhaltenen Strom (100) weiter zu destillieren. Dies geschieht bevorzugt in einem Schritt B.V):
V) Gewinnung eines flüssigen Isocyanat-Stroms (140) aus dem flüssigen Strom (100), wobei ein Nebenkomponenten und gegebenenfalls Lösungsmittel enthaltender gasförmiger Strom (150) anfällt, in einer Destillationsvorrichtung (2400), optional umfassend die Abtrennung von polymeren Isocyanatfraktionen in einer vorgeschalteten Einrichtung zur Polymerabtrennung (2410) als Strom (141).

Der Strom (140) umfasst also in dieser Ausführungsform das Isocyanat (1) in einer gereinigten Form. Dabei kann (140) auch summarisch für verschiedene Isocyanat-Ströme (1) *unterschiedlicher Isomerenzusammensetzung* (140-1, 140-2, ...) stehen, sofern die Destillation in 2400 nicht nur eine Reinigung, sondern auch eine Isomerentrennung beinhaltet. Daneben enthält auch der in besonderen Ausführungsformen anfallende Strom 141 Isocyanate (1), wobei Strom 141 vor allem *polymere Isocyanatfraktionen* (das sind Isocyanate (1), die sich von polymerisierten Aminen ableiten lassen, z. B. Polymethylenpolyphenylenpolyisocyanate mit drei oder mehr Benzol-"Kernen") umfasst. Auch der in besonderen Ausführungsformen - d. h. insbesondere bei Verzicht auf die vorgeschaltete Polymerabtrennung in 2410 - anfallende "Rückstandsstrom" 143 enthält noch Isocyanat (1), welches aus diesem Strom gewonnen werden kann. Die letztgenannte Ausführungsform eignet sich besonders für die Herstellung von Toluylendiisocyanat, wobei dann die Destillationsvorrichtung 2400 vorzugsweise als Trennwandkolonne ausgestaltet wird.

Schritt V) kann nach einem beliebigen aus dem Stand der Technik bekannten Verfahren erfolgen. Geeignete Verfahren sind beschrieben in EP1475367B1 oder auch in EP 1 371 635 B1.

Im erfindungsgemäßen Verfahren werden insbesondere Einrichtungen der Verfahrensschritte III), IV) und V) [(d. h. (2200), (2300), sofern vorhanden (2410) und (2400)] bei einem gegenüber Atmosphärendruck vermindertem Druck betrieben. Die hierfür erforderlichen Vakuumerzeugungsanlagen produzieren Abgasströme (in FIG. 1 nicht gezeigt). Diese Abgasströme werden im Stand der Technik üblicherweise, bevorzugt nach Vereinigung, bei vergleichsweise niedriger Temperatur (-30,0 °C bis 0,0 °C) partiell kondensiert, um mitgerissenes Lösungsmittel auszukondensieren. Das erfindungsgemäße Verfahren ermöglicht die Durchführung dieser Kondensation bei höherer Temperatur (1,0 °C bis 50,0 °C; Einsparung von Kälteenergie) oder sogar den völligen Verzicht auf diese Kondensation. Es ist erfindungsgemäß auch möglich (und bevorzugt), anstelle von besagter Kondensation eine einfache Flüssigkeitsabscheidung (Tropfenabscheidung) durchzuführen. Geeignete Einrichtung hierfür sind dem Fachmann bekannt und umfassen bevorzugt Schwerkraftabscheider, Zyklone, Lamellenabscheider, Gestricke und dergleichen mehr. Das so erhaltene von mitgerissener Flüssigkeit durch Kondensation bei vergleichsweise hoher Temperatur oder Flüssigkeitsabscheidung befreite Abgas der Vakuumerzeugungsanlagen kann grundsätzlich direkt dem Schritt a) zugeführt werden. Es ist jedoch bevorzugt, diesen Strom ebenso wie den Strom (200) vorher noch weiter zu reinigen und insbesondere Spuren an Phosgen zu zersetzen. Zu diesem Zweck wird das von mitgerissener Flüssigkeit befreite Abgas der Vakuumerzeugungsanlagen in der Vorrichtung zur Aufarbeitung von Abgasströmen (3000) gereinigt, wobei die Abgasströme ebenso wie Strom (200) zunächst in eine Vorrichtung zur Phosgenzersetzung (3010; "Phosgenvernichtung") geleitet und anschließend als Bestandteil des gasförmigen Stroms (210) in die Adsorptionsvorrichtung (3020) zur Adsorption von Lösungsmittel aus Schritt a) geführt werden.

Ein weiterer Gegenstand der vorliegenden Erfindung ist eine **Anlage (10000) zur Herstellung eines Isocyanats (1) durch Phosgenierung des korrespondierenden Amins (2),** umfassend die Anlagenteile
0) optional und bevorzugt, eine Vorrichtung (4000) zur Erzeugung von Phosgen (3) durch Umsetzung von Kohlenstoffmonoxid (300) mit Chlor (310), wobei ein Kohlenstoffmonoxid und Phosgen enthaltender Abgasstrom (320) anfällt;
1) eine Reaktionsstrecke (1000) umfassend einen Reaktionsraum (1200) zur Durchführung der Phosgenierung, dem optional eine Abscheideeinrichtung (1210) nachgeschaltet ist, wobei der Reaktionsraum oder die Abscheideeinrichtung mit Abfuhrleitungen für einen flüssigen Strom (60) und einen gasförmigen Strom (70) versehen sind;
II) eine Destillationsvorrichtung (2100) zur Auftrennung des flüssigen Stroms (60) in einen flüssigen Strom (80) und einen gasförmigen Strom (90);
III) optional und bevorzugt, eine Destillationsvorrichtung (2200) zur Trennung des flüssigen Stroms (80) in einen gasförmigen Strom (110) und einen flüssigen Strom (100);
IV) optional und bevorzugt, eine Destillationsvorrichtung (2300) zur Trennung des gasförmigen Stroms (110), bevorzugt nach dessen Verflüssigung in einem Kondensator (2310), in einen flüssigen Strom (120) und einen gasförmigen Strom (130);
V) optional und bevorzugt, eine Destillationsvorrichtung (2400) zur Gewinnung eines flüssigen Isocyanat-Stroms (140) aus dem flüssigen Strom (100), wobei ein Nebenkomponenten und gegebenenfalls Lösungsmittel enthaltender gasförmiger Strom (150) anfällt, optional umfassend eine vorgeschaltete Einrichtung zur Polymerabtrennung (2410) zur Abtrennung von polymeren Isocyanatfraktionen (141);
VI) eine Vorrichtung zur Absorption (2500) der gasförmigen Ströme (70) und (90) und, sofern vorhanden, des gasförmigen Stroms (130), gegebenenfalls nach Durchlaufen von der Vorrichtung zur Absorption (2500) vorgeschalteten Einrichtungen (2510) zur Kondensation (in FIG. 1 nicht eingezeichnet), in Lösungsmittel (4) unter Erhalt eines flüssigen Stroms (160) und eines gasförmigen Stroms (170);
VII) eine Vorrichtung zur Absorption (2600) des gasförmigen Stroms (170) in Wasser oder Salzsäure, bevorzugt umfassend einen Brüdenkondensator (2610), unter Erhalt eines Salzsäure enthaltenden Stroms (190) und eines gasförmigen, Lösungsmittel und gegebenenfalls gasförmige Nebenkomponenten enthaltenden Stroms (200);
VIII) eine Vorrichtung zur Aufarbeitung von Abgasströmen (3000), umfassend
   VIII-1) eine Vorrichtung zur katalytischen Phosgenzersetzung (3010) zur Zersetzung von in Strom (200) enthaltenen Phosgens und bevorzugt auch zur Zersetzung von in Strom (320) enthaltenen Phosgens unter Gewinnung eines gasförmigen, Lösungsmittel und gegebenenfalls gasförmige Nebenkomponenten enthaltenden Stroms (210);
   VIII-2) eine Adsorptionsvorrichtung (3020) zur Adsorption von Lösungsmittel aus dem Strom (210), wobei die Adsorptionsvorrichtung (3020) mindestens zwei parallel geschaltete Adsorptionseinrichtungen (3021, 3022) umfasst, die so ausgestaltet sind, dass diese wechselweise
      (i) mit dem Strom (210) beaufschlagt und
      (ii) mit Wasserdampf regeneriert werden können;
IX) eine Vorrichtung zur Abgasverbrennung (6000) zur Verbrennung des in VIII-2) (i) erhaltenen Gasstroms (220);
X) Einrichtungen zur Rezyklierung von in Verfahrensstufe VIII-2) (ii) desorbierten Lösungsmittels in die Reaktionsstrecke (1000) und/oder in die Vorrichtung zur Absorption (2500).

Durch das erfindungsgemäße Verfahren ergeben sich mindestens die folgenden **Vorteile:**
i) Lösungsmittel kann aus dem der Verbrennung zuzuführenden Abgasstrom ohne energieintensive Abkühlung auf sehr tiefe Temperaturen (< -30,0 °C) zurückgewonnen werden. Instandhaltungskosten für eine Tiefkälteerzeugung entfallen.
ii) Minimierung des in der Abgasverbrennung verbrannten Lösungsmittelanteils, dadurch Minimierung von Verbrennungsrückständen. Da in der Isocyanatherstellung eingesetzte Lösungsmittel häufig chlorhaltig sind, kann darüber hinaus die Reinigung der Verbrennungsabgase vereinfacht werden.
iii) Durch die Rückgewinnung des Lösungsmittels wird der Zukauf an frischem Lösungsmittel drastisch reduziert, dadurch schonenderer Umgang mit Ressourcen.
iv) Einsparung einer elektrischen Begleitheizung auf der Abgasleitung zwischen Phosgenzersetzung und Abluftverbrennung und eines Flüssigkeitsabscheiders in der Abgasleitung.

Der Erfolg der erfindungsgemäßen Vorgehensweise zur Lösungmittelrückgewinnung durch Einsatz einer nachgeschalteten Lösungsmitteladsorptionsanlage gegenüber einer reinen thermischen Verwertung des Lösungsmittels in der Verbrennung waren trotz der Investitionskosten für eine zusätzliche Adsorptionsstufe für den Fachmann überraschend. Eine Produktionsanlage mit Lösungsmitteladsorption ist mit geringem Dampfverbrauch und sehr geringem Instandhaltungsaufwand (Wechsel der Aktivkohle der Adsorption häufig erst nach fünf Jahren erforderlich) zu betreiben.

Im Folgenden soll die vorliegende Erfindung durch konkrete Beispiele weiter verdeutlicht werden.

### Beispiele

### Allgemeine Bedingungen für die Herstellung eines Gemisches aus Methylendiphenylendiisoyanat und Polymethylenpolyphenylenpolyisocyanat (nachfolgend summarisch MDI) im Regelbetrieb - Verfahrensstufen I bis IX; vgl. auch FIG. 1

20,4 t/h eines Gemisches aus Methylendiphenylendiamin und Polymethylenpolyphenylenpolyamin (nachfolgend summarisch MDA; 2) einer Temperatur von 110,0 °C werden mit 55,0 t/h Monochlorbenzol (MCB; 4) einer Temperatur von 30,0 °C als Lösungsmittel über einen statischen Mischer (1020) zu einer 27,1%igen MDA-Lösung (20) vermischt. Phosgen (3) wird bereitgestellt über eine Vorrichtung zur Erzeugung von Phosgen (4000) umfassend zwei Phosgengeneratoren und zwei Phosgenverflüssiger (siehe die Beschreibung der Verfahrensstufe 0 weiter unten). Im Anschluss wird das Phosgen (3) in einem Phosgenlösungstank (1030) mit Strom 160, der überwiegend aus MCB (4) besteht, zu einer 35,0%igen Phosgenlösung (30) vermischt. Stündlich werden 120 Tonnen dieser Phosgenlösung (30) einer Temperatur von 0,0 °C mit 20,4 Tonnen 27,1%iger MDA-Lösung (20) einer Temperatur von 45,0 °C in einer adiabaten Reaktion, wie in EP 1 873 142 B1 beschrieben, umgesetzt. Nach der Durchmischung der beiden Rohstofflösungen im Mischaggregat (1100) wird die erhaltene Reaktionslösung (50) mit einer Temperatur von 81,0 °C über eine Rohrleitung (sog. "Suspensionsleitung") in einen beheizten Phosgenierturm (1200) gefahren. Am Kopf des Phosgenierturmes liegen ein absoluter Druck von 1,6 bar und eine Temperatur von 111,0 °C vor. Der bei der Reaktion entstandene Chlorwasserstoff wird zusammen mit Spuren an Phosgen und MCB als Gasstrom (70) abgeführt. Das flüssige Reaktionsgemisch (60) wird dem Phosgenierturm (1200) entnommen und der Aufarbeitungssequenz (II ff.) zugeführt. Dazu wird es zunächst als Seitenstrom in eine beheizte Entphosgenierkolonne (2100, II) eingeleitet. Bei einer Kopftemperatur von 116,0 °C und einem absoluten Druck von 1,6 bar wird über Kopf Phosgen mit Spuren MCB und Chlorwasserstoff abgeführt (90). Phosgen wird in einer Phosgenabsorptionskolonne (2500, VI) im MCB absorbiert, und die erhaltene Phosgenlösung wird in den Phosgenlösungstank (1030) gefahren. Chlorwasserstoff wird in einen Chlorwasserstoffabsorber (2600, VII) geführt; die dort erhaltene Salzsäure wird zur weiteren Verwendung in den Salzsäuretank (2630) geleitet. Die Brüden der Chlorwasserstoffabsorption (2600) werden im Kondensator 2610 partiell verflüssigt; der verbleibende Gasstrom (200) wird der Vorrichtung zur Aufarbeitung von Abgasströmen (3000, VIII) zugeführt, die mindestens eine Phosgenzersetzung (3010) als in Strömungsrichtung erste Einrichtung umfasst. Der Abgasweg ist ausgehend von der gasförmigen Phase der Phosgenabsorptionskolonne (2500, VI) in die Chlorwasserstoff-Absorption (2600, VII) und von dort über die gasförmige Phase in die Vorrichtung zur Aufarbeitung von Abgasströmen (3000, VIII) bis zur Abgasverbrennung (6000, IX) geöffnet.

Nach Abtrennung von Chlorwasserstoff und überschüssigem Phosgen aus der Isocyanat enthaltenden Reaktionslösung (60) wird eine Isocyanat-Rohlösung (80) erhalten, die aus dem Sumpf der Entphosgenierkolonne 2100 ausgetragen und mit einer Temperatur von 155,0 °C in eine erste Destillationsstufe der Lösungsmitteldestillation (2200, III) gefahren wird, um sie vom Lösungsmittel MCB zu befreien. Der absolute Druck am Kopf dieser Lösungsmitteldestillationskolonne (2200) beträgt 600 mbar bei einer Sumpftemperatur von 145,0 °C. Über Kopf wird MCB gasförmig abgezogen (110), wobei dieser MCB-Gasstrom in einem Luftkühler (2310) kondensiert wird. 18,0 t/h dieses kondensierten Lösungsmittels werden in eine Waschkolonne (nicht gezeigt in FIG. 1) gesprüht, um einen möglichen Mitriss von Isocyanat in die Vakuumleitungen zu verhindern. Das restliche kondensierte MCB von 100 t/h wird zu einer Destillationskolonne (2300, IV) gepumpt, in dem das MCB von Phosgen befreit wird, wobei die Phosgen-haltigen Brüden (130) kondensiert und in den Phosgenabsorber (2500, VI) gepumpt werden, und das Phosgen-freie MCB aus dem Sumpf des Lösungsmittelstrippers (120) in den Lösungsmitteltank (1040) gepumpt wird. Das Roh-MDI wird aus dem Sumpf der Kolonne 2200 ausgetragen und in einer zweiten Destillationskolonne (nicht gezeigt in FIG. 1) bis auf 1 % vom restlichen MCB befreit. Der absolute Druck am Kopf dieser Lösungsmitteldestillationskolonne beträgt 70 mbar bei einer Sumpftemperatur von 150,0 °C. Über Kopf wird MCB gasförmig abgezogen, wobei dieser MCB-Gasstrom kondensiert wird und in den Sumpf der ersten Destillationskolonne (2200) zurückgeführt wird. Anschließend wird in einem Gegenstromverdampfer bei einem absoluten Druck von 20 mbar und einer Kopftemperatur von 170,0 °C das Produkt von Nebenkomponenten wie Phenylisocyanat und restlichem MCB befreit. Dabei fallen als Sumpfprodukt (100) 25,45 t/h MDI an, das mittels weiterer Destillationsschritte (2410 / 2400) in Methylendiphenyldiisoyanat (140) und ein Gemisch aus Methylendiphenyldiisoyanat und Polymethylenpolyphenylpolyisocyanat (141) getrennt wird.

### Allgemeine Bedingungen für die Herstellung von Phosgen - Verfahrensstufe 0

In einem Mischrohr werden kontinuierlich 4400 Nm³/h Chlor und 4650 Nm³/h Kohlenmonoxid bei 18,0 °C und einem Druck von 1,8 bar (absolut) gemischt. Es wird ein Überschuss an Kohlenmonoxid, bezogen auf Chlor, eingesetzt, sodass nach der vollständigen Umsetzung des Chlors noch 9,0 % Kohlenmonoxid im Phosgen verbleiben. Das Mischgas aus Chlor und Kohlenmonoxid wird in einen am Boden befindlichen Verteiler eines Rohrbündel-Phosgengenerators gefahren. In den Rohren über dem Verteiler befinden sich als Katalysator 10 Tonnen Aktivkohle (Norit RB4C). An diesem Katalysator reagiert das Mischgas in einer stark exothermen Reaktion zu Phosgen ab. Die Reaktion wird über einen Wasserkreislauf mittels Wassersiedekühlung gekühlt. Die Temperatur des Phosgens in der Austrittsleitung des Generators beträgt 55,0 °C und der Druck liegt bei 1,53 bar (absolut). An diesem Punkt wird die Vollständigkeit der Reaktion überwacht, indem der Restchlorgehalt und der Kohlenmonoxidgehalt kontinuierlich gemessen werden. Das so hergestellte, gasförmige, überschüssiges Kohlenmonoxid enthaltende Phosgen wird dann in einem Phosgenverflüssiger bei -17,0 °C kondensiert. Das Sumpfprodukt aus dem Phosgenverflüssiger läuft in einen Phosgenlösungstank (1030). Überschüssiges Kohlenmonoxid kondensiert nicht und wird über Kopf in einen nachgeschalteten baugleichen zweiten Phosgengenerator gefahren und dort mit einer entsprechenden Menge an Chlor beaufschlagt, sodass wiederum nach vollständiger Umsetzung des Chlors noch 9,0 % an Kohlenmonoxid im Phosgen verbleiben. Auch nach dem zweiten Phosgengenerator wird die Vollständigkeit der Reaktion überwacht, indem der Restchlorgehalt und der Kohlenmonoxidgehalt kontinuierlich gemessen werden. Das so hergestellte Phosgen wird in einem zweiten Phosgenverflüssiger bei -17,0 °C kondensiert. Das Sumpfprodukt aus dem zweiten Phosgenverflüssiger läuft ebenfalls in den Phosgenlösungstank. Somit kommen pro Stunde 42 Tonnen Phosgen im Phosgenlösungstank an.

Als Kopfprodukt (Strom 320) des Phosgenverflüssigers werden 150 m³/h überschüssiges Kohlenmonoxid, dem auch Spuren an Phosgen (0,50 % der Gesamtabgasmenge) anhaften, in einer Absorptionskolonne (sog. Phosgenwäscher (4010)), die mit -17,0 °C kaltem Lösungsmittel (MCB) betrieben wird, vorgereinigt, wobei ein Teil des Phosgens aus dem Gasstrom herausgewaschen wird. Die so erhaltene phosgenhaltige MCB-Lösung wird der Phosgenabsorption (2500) als flüssiger Strom (340) zugeführt. Der restliche Abgasstrom (330), dem noch Spuren an Lösungsmittel und Phosgen anhaften, wird mit Strom (200) vereint und der Phosgenzersetzung (3010) zugeführt.

Im Phosgenlösungstank (1030) kann das Phosgen bei Bedarf mit weiterem Lösungsmittel (4) vermischt werden (über den Strom 160 ist im Phosgenlösungstank immer Lösungsmittel vorhanden).

Zur Kälteerzeugung in den Phosgenverflüssigern werden sog. Ammoniakschraubenverdichter eingesetzt. Damit wird das Lösungsmittel MCB, das in der Reaktion Verwendung findet, und das Kühlmittel MCB, mit dem die Kühler für die Kondensation der Abgasströme versorgt werden, auf -17,0 °C abgekühlt.

### Beispiel 1 (Vergleichsbeispiel): teilweise Rückgewinnung des Lösungsmittels unter Einsatz von MCB einer Temperatur von -17,0 °C für die Kondensation des Abgases der Vakuumerzeugung

Die Anlagen zur Herstellung von Phosgen und zur Herstellung von MDI wurden, wie in den beiden allgemeinen Herstellungsbedingungen beschrieben, bei Nennlast betrieben. In allen Abgasströmen der beiden Anlagen wurde MCB mitgeführt. Die Abgaströme setzten sich zusammen aus den Abgasen der Phosgenherstellung in Verfahrensstufe 0) (150 m³/h), der Vakuumerzeugung der Destillation in Verfahrensschritt V) (50 m³/h) und der Chlorwasserstoffabsorption in Verfahrensstufe VII) (100 m³/h). Das Abgas der Vakuumerzeugung (in den Zeichnungen nicht eingezeichnet) von den Vakuumpumpen und deren Vorlagen der MDI-Anlage der Verfahrensschritte III, IV und V wurde vereint und über einen Kondensator, der mit MCB einer Temperatur von -17,0 °C betrieben wurde, der Phosgenzersetzung (3010) zugeführt. Der Abgasstrom (170) der Phosgenabsorption (7500 m³/h) wurde in den Chlorwasserstoffabsorber (2600) geleitet. Zur Rückgewinnung des in den Abgasströmen vorhandenen Lösungsmittels MCB wurde wie folgt vorgegangen. Die 150 m³/h des Abgasstroms (330) aus dem Phosgenwäscher (4010) der Phosgenanlage wurden mit den 100 m³/h des Abgasstroms (200) aus dem Kondensator der Chlorwasserstoffabsorption (2610) der MDI-Anlage vereint und der Phosgenzersetzung (3010) zugeführt. Das vereinte Abgas wurde mittels eines Ventilators, der hinter der Phosgenzersetzung angebracht ist, durch diese gefahren. Bei Nennlast wurden insgesamt 300 m³/h an Abgas in die Phosgenzersetzung (3010) gefahren. Die zwei mit jeweils 14 m³ Aktivkohle Norit RB4C befüllten hintereinandergeschalteten Phosgenvernichtungstürme wurden im Unterdruck bei 930 mbar absolut betrieben. Die Phosgenzersetzung wurde mit einem Kreislauf von 25,0 °C kaltem Kondensat (Strom 260) betrieben, das die Aktivkohle permanent benetzte und dünne Salzsäure (Strom 270) auswusch, die in den Dünnsäuretank (2640) gefahren wurde. Der die Phosgenzersetzung verlassende Abgasstrom (210) von 300 m³/h wurde der Verbrennung (6000) zugeführt. Die Abgasleitung zwischen Phosgenzersetzung und der Abgasverbrennung ist begleitbeheizt, um Kondensation von MCB zu minimieren. Zusätzlich ist ein Flüssigkeitsabscheider in diese Abgasleitung eingebaut, um kondensiertes, wasserfeuchtes, saures MCB abzulassen.

**Bilanz:** Insgesamt gingen so bei 8000 Betriebsstunden 330 Tonnen an Lösungsmittel pro Jahr über die Abgasverbrennung verloren, und weitere 100 Tonnen an Lösungsmittel pro Jahr, die aus dem Flüssigkeitsabscheider abgelassen wurden, mussten separat verbrannt werden. Für die Kälteenergie zum Auskondensieren des Lösungsmittels aus dem Abgas der Vakuumerzeugung wurden 103 kWh benötigt.

### Beispiel 2 (erfindungsgemäß): Rückgewinnung des Lösungsmittels mittels einer Vorrichtung zur Lösungsmitteladsorption ohne Einsatz von MCB einer Temperatur von -17,0 °C zur Kondensation von MCB im Abgas

Die Anlagen zur Herstellung von Phosgen und zur Herstellung von MDI wurden, wie in den beiden allgemeinen Herstellungsbedingungen beschrieben, bei Nennlast betrieben. In allen Abgasströmen der beiden Anlagen wurde MCB mitgeführt. Die Abgaströme setzten sich zusammen aus den Abgasen der Phosgenherstellung in Verfahrensstufe 0) (150 m³/h), der Vakuumerzeugung der Destillation in Verfahrensschritt V) (50 m³/h) und der Chlorwasserstoffabsorption in Verfahrensstufe VII) (100 m³/h). Die Abgasströme der Vakuumerzeugung (in den Zeichnungen nicht eingezeichnet) von den Vakuumpumpen und deren Vorlagen der Verfahrensschritte III, IV und V wurden vereint und bei 29,0 °C über einen Flüssigkeitsabscheider (in dem MCB- und Phosgen-haltige Tröpfchen, die im Abgas mitgeführt wurden, durch Schwerkraft abgeschieden wurden) der Phosgenzersetzung (3010) zugeführt. Der Abgasstrom (170) der Phosgenabsorption (9000 m³/h) wurde in den Chlorwasserstoffabsorber (2600) geleitet. Zur Rückgewinnung des in den Abgasströmen vorhandenen Lösungsmittels MCB wurde wie folgt vorgegangen. Die 150 m³/h des Abgasstroms (330) aus dem Phosgenwäscher (4010) der Phosgenanlage wurden mit den 100 m³/h des Abgasstroms (200) aus dem Kondensator der Chlorwasserstoffabsorption (2610) der MDI-Anlage vereint und der Phosgenzersetzung (3010) zugeführt. Das vereinte Abgas wurde mittels eines Ventilators, der hinter der Phosgenzersetzung angebracht ist, durch diese gefahren. Bei Nennlast wurden insgesamt 300 m³/h an Abgas in die Phosgenzersetzung (3010) gefahren. Die zwei mit jeweils 14 m³ Aktivkohle Norit RB4C befüllten hintereinandergeschalteten Phosgenvernichtungstürme wurden im Unterdruck bei 900 mbar absolut betrieben. Die Phosgenzersetzung wurde mit einem Kreislauf von 25,0 °C kaltem Kondensat (Strom 260) betrieben, das die Aktivkohle permanent benetzte und dünne Salzsäure (Strom 270) auswusch, die in den Dünnsäuretank (2640) gefahren wurde. Der die Phosgenzersetzung verlassende Abgasstrom (210) von 300 m³/h wurde durch die Lösungsmitteladsorption (3020) geleitet.

Die Lösungsmitteladsorption wurde so betrieben, dass das Lösungsmittel-haltige Abgas aus Strom (210) bei 47,0 °C, über 2 m³ Aktivkohle Norit RB4C geleitet wurde, wobei das Lösungsmittel an der Aktivkohle bei 950 mbar absolut angelagert wurde. Der Abgasstrom (220) wurde kontinuierlich mit einem Analysator (FID) auf organischen Kohlenstoff überwacht. Das Beladungsintervall wurde so getaktet, dass es keine Lösungsmittelverluste gab. Um Lösungsmittelverluste infolge Sättigung der Aktivkohle mit Lösungsmittel über den Abgasstrom (220) zu vermeiden, wurde nach einem zweistündigen Beladungsintervall die Aktivkohle für eine Stunde regeneriert. Die Lösungsmitteladsorption wurde während dieser Zeit über eine parallel geschaltete, identisch aufgebaute Lösungsmitteladsorption weitergeführt. Die Regeneration der Aktivkohle wurde so durchgeführt, dass das auf der Aktivkohle adsorbierte Lösungsmittel für eine Stunde mit 0,15 Tonnen direktem 2,5-bar-Dampf desorbiert wurde. Das dabei entstehende heiße, wässrige Lösungsmittelgemisch (230) wurde mittels eines Kondensators (5010), der mit Kühlwasser betrieben wurde, auf 30,0 °C abgekühlt und in einem Lösungsmittelabscheider (5020) in eine wässrige Phase (250), die in den Dünnsäuretank (2640) geleitet wurde, und in eine organische Phase (240), enthaltend das mit Wasser gesättigte Lösungsmittel MCB, das mit 100 kg/h in einen Vorlagebehälter (2700) geleitet wurde, getrennt. Die Lösungsmitteltrocknung wurde diskontinuierlich zweimal pro Woche in Betrieb genommen. Die Trocknung erfolgte mit einem Molsieb mit einer Porenweite von 4 Ǻ. Dazu wurden 5 Tonnen feuchtes Lösungsmittel MCB mit einer Pumpe aus dem Vorlagebehälter (2700) während 15 Stunden von unten nach oben durch einen Trocknungsbehälter (2710), der mit 50 kg des Molsiebes befüllt war, geleitet. Das den Trocknungsbehälter verlassende MCB hatte noch eine Restfeuchte von 40 ppm und wurde in die Lösungsmitteldestillation (2200) rezykliert. Das Molsieb wurde anschließend mit 20 m³/h Stickstoff einer Temperatur von 80,0 °C über zwei Tage regeneriert. Der Stickstoff wurde über einen Wärmeaustauscher mit 2,5 bar überschüssigem, selbsterzeugtem Dampf aufgeheizt.

**Bilanz:** Insgesamt gingen so bei 8000 Betriebsstunden 30 Tonnen an Lösungsmittel pro Jahr verloren. Kälteenergie zum Auskondensieren des Lösungsmittels aus dem Abgas der Vakuumerzeugung wurde nicht benötigt. Für die Regeneration des Molsiebes der Lösungsmitteltrocknung wurden 20.000 m³/a heißer Stickstoff verbraucht, der mit überschüssigem selbsterzeugten 2,5 bar Dampf erhitzt wurde. Das Molsieb muss einmal pro Jahr erneuert werden. Für die Regeneration der Aktivkohle der Lösungsmitteladsorption wurden 600 to/a an überschüssigem, selbsterzeugten 2,5 bar Dampf verbraucht. Die Aktivkohle der Lösungsmitteladsorption muss alle 5 Jahre erneuert werden.

**Fazit:** Insgesamt werden erfindungsgemäß 400 Tonnen MCB und 828 KW Kälteenergie eingepart. Dem gegenüber stehen zusätzliche Aufwendungen für Apparate (Lösungsmitteladsorption, - kondensator, -abscheider, -trocknung, Vorlagen und Rohrleitungen) sowie für den Hilfsstoff Stickstoff. Das Betreiben dieser zusätzlichen Einrichtungen ist jedoch mit nur äußerst geringen Investitionskosten und Instandhaltungskosten behaftet. Die Kosten für Molsieb und Aktivkohle sowie für Stickstoff sind gering. Der benötigte Dampf ist selbsterzeugter, überschüssiger Dampf, der ansonsten über einen Luftkühler kondensiert und der Kondensatweiterverarbeitung zugeführt werden müsste.

## Patentansprüche

1. Verfahren zur Herstellung eines Isocyanats (1), umfassend die Schritte:
A. Umsetzung des zu dem Isocyanat (1) korrespondierenden Amins (2) mit Phosgen (3) unter Einsatz eines Lösungsmittels (4), wobei ein das Isocyanat (1) und Lösungsmittel (4) enthaltender flüssiger Strom sowie mindestens ein lösungsmittelhaltiger Abgasstrom erhalten werden;
B. Aufarbeitung des Isocyanat und Lösungsmittel enthaltenden flüssigen Stroms zur Isolierung des Isocyanats, wobei weiteres Lösungsmittel (4) eingesetzt werden kann, wobei ebenfalls mindestens ein lösungsmittelhaltiger Abgasstrom erhalten wird;
wobei
a) der mindestens eine lösungsmittelhaltige Abgasstrom aus Schritt A und/oder aus Schritt B in eine Adsorptionsvorrichtung zur Adsorption von Lösungsmittel (3020) geführt wird, welche mindestens zwei parallel geschaltete Adsorptionseinrichtungen (3021, 3022) umfasst, die wechselweise
(i) mit dem mindestens einen Abgasstrom beaufschlagt und
(ii) mit Wasserdampf regeneriert werden,
wobei während der Verfahrensstufe a) (i) ein an Lösungsmittel abgereicherter Abgasstrom (220) erhalten wird und während der Verfahrensstufe a) (ii) ein Strom enthaltend Wasser und Lösungsmittel (230) erhalten wird und
b)
(i) der an Lösungsmittel abgereichterte Abgasstrom (220) einer Abgasverbrennung (6000) zugeführt wird und
(ii) der Strom enthaltend Wasser und Lösungmittel (230) an Wasser abgereichert und danach in Schritt A und/oder in Schritt B zurückgeführt wird.

2. Verfahren gemäß Anspruch 1, bei welchem Schritt A umfasst:
I) Umsetzung des Amins (2) mit Phosgen (3) und Trennung des erhaltenen Verfahrensprodukts in den das Isocyanat und Lösungsmittel enthaltenden flüssigen Strom (60) und einen gasförmigen, Phosgen, Chlorwasserstoff und Lösungsmittel enthaltenden Strom (70);
und bei welchem Schritt B umfasst:
II) Abreicherung von Phosgen und Chlorwasserstoff aus dem flüssigen Strom (60) aus Schritt I) durch Auftrennung dieses flüssigen Stroms (60) in einen flüssigen, Lösungsmittel und Isocyanat enthaltenden Strom (80) und einen gasförmigen, Phosgen und Chlorwasserstoff enthaltenden Strom (90) in einer Destillationsvorrichtung (2100);
wobei die gasförmigen Ströme (70) und (90), optional nach Durchlaufen weiterer Reinigungsschritte, als Bestandteil, gegebenenfalls als alleiniger Bestandteil, des mindestens einen lösungsmittelhaltigen Abgasstroms dem Schritt a) zugeführt werden.

3. Verfahren gemäß Anspruch 2, bei welchem sich an Schritt B.II) anschließt:
III) Abreicherung von Lösungsmittel aus dem flüssigen Strom (80) aus Schritt II) durch Trennung dieses flüssigen Stroms (80) in einen gasförmigen, Lösungsmittel enthaltenden Strom (110) und eine flüssigen, Isocyanat enthaltenden Strom (100) in einer Destillationsvorrichtung (2200);
IV) Abreicherung von Phosgen aus dem gasförmigen Strom (110) aus Schritt III) durch Trennung dieses gasförmigen Stroms (110), optional nach dessen Verflüssigung in einem Kondensator (2310), in einen flüssigen, Lösungsmittel enthaltenden Strom (120) und einen gasförmigen, Phosgen enthaltenden Strom (130) in einer Destillationsvorrichtung (2300);
wobei der gasförmige Strom (130), optional nach Durchlaufen weiterer Reinigungsschritte, als Bestandteil des mindestens einen lösungsmittelhaltigen Abgasstroms dem Schritt a) zugeführt wird.

4. Verfahren gemäß Anspruch 3, bei welchem sich an Schritt B.IV) anschließt:
V) Gewinnung eines flüssigen Isocyanat-Stroms (140) aus dem flüssigen Strom (100), wobei ein Nebenkomponenten und gegebenenfalls Lösungsmittel enthaltender gasförmiger Strom (150) anfällt, in einer Destillationsvorrichtung (2400), optional umfassend die Abtrennung von polymeren Isocyanatfraktionen in einer vorgeschalteten Einrichtung zur Polymerabtrennung (2410) als Strom (141).

5. Verfahren gemäß einem der Ansprüche 2 bis 4, bei welchem Schritt B zusätzlich umfasst:
VI) Reinigung der gasförmigen Ströme (70) und (90) und, sofern vorhanden, des gasförmigen Stroms (130), gegebenenfalls nach Kondensation, durch Absorption in Lösungsmittel (4) unter Erhalt eines flüssigen, Lösungsmittel und Phosgen enthaltenden Stroms (160) und eines gasförmigen, Chlorwasserstoff und Lösungsmittel enthaltenden Stroms (170) in einer Absorptionsvorrichtung (2500).

6. Verfahren gemäß Anspruch 5, bei welchem sich an Schritt B.VI) anschließt:
VII) Reinigung des gasförmigen Stroms (170) in Wasser oder Salzsäure als Absorptionsmittel (180) in einer weiteren Absorptionsvorrichtung (2600), wobei ein Salzsäure enthaltender Strom (190) und, optional nach Durchlaufen eines Brüdenkondensators (2610), ein gasförmiger, Lösungsmittel und gegebenenfalls gasförmige Nebenkomponenten enthaltender Strom (200) erhalten werden.

7. Verfahren gemäß Anspruch 6, bei welchem in Schritt B.VII) der Brüdenkondensator (2610) durchlaufen wird und dieser Schritt zusätzlich umfasst:
Trennung des im Brüdenkondensator (2610) erhaltenen flüssigen Stroms (191) in eine wässrige Phase (192) und eine organische Phase (193);
Rezyklierung der wässrigen Phase (192) als Bestandteil des Absorptionsmittels (180) in die Absorptionsvorrichtung (2600) und/oder
Rezyklierung der organischen Phase (193), optional nach Trockung, in mindestens einen der Schritte A.I), B.III), B.IV und B.VI).

8. Verfahren gemäß einem der Ansprüche 6 oder 7, bei welchem sich an Schritt B.VII) anschließt:
VIII) Reinigung des gasförmigen, Lösungsmittel und gegebenenfalls gasförmige Nebenkomponenten enthaltenden Stroms (200) in einer Vorrichtung zur Aufarbeitung von Abgasströmen (3000), wobei
VIII-1) der aus der Absorptionsvorrichtung (2600), optional nach Durchlaufen des Brüdenkondensator (2610), stammende gasförmige Strom (200) in eine Vorrichtung zur Phosgenzersetzung (3010) geleitet wird, in welcher Phosgen katalytisch unter Verwendung von Wasser (260) zersetzt wird, wobei ein gasförmiger, Lösungsmittel und gegebenenfalls gasförmige Nebenkomponenten enthaltender Strom (210) und ein salzsaurer flüssiger Strom (270) erhalten werden, wobei der gasförmige Strom (210) in die Adsorptionsvorrichtung (3020) zur Adsorption von Lösungsmittel in Schritt a) geführt wird.

9. Verfahren gemäß einem der vorstehenden Ansprüche, insbesondere gemäß Anspruch 8, bei welchem
das Phosgen (3) für die Durchführung von Schritt A durch Umsetzung von Kohlenstoffmonoxid (300) mit Chlor (310) hergestellt wird, wobei ein Kohlenstoffmonoxid und Phosgen enthaltender Abgasstrom (320) anfällt, der, optional nach Durchlaufen weiterer Reinigungsschritte, dem der Abgasverbrennung zuzuführenden mindestens einen lösungsmittelhaltigen Abgasstrom zugeführt wird.

10. Verfahren gemäß Anspruch 9, bei welchem der Kohlenstoffmonoxid und Phosgen enthaltende Abgasstrom (320) aus der Umsetzung von Kohlenstoffmonoxid (300) mit Chlor (310) in Lösungsmittel (4) einer Temperatur im Bereich von 0,0 °C bis -20,0 °C in einer Absorptionskolonne (4010) zu einem an Phosgen abgereicherten Abgasstrom (330) gereinigt wird, welcher vor Durchführung von Schritt VIII.1) mit dem gasförmigen Strom (200) vereinigt wird, wobei in der Absorptionskolonne (4010) ein Lösungsmittel- und Phosgen-haltiger Strom (340) anfällt, der der Absorptionsvorrichtung (2500) zugeführt wird.

11. Verfahren gemäß einem der vorstehenden Ansprüche, bei welchem die Abreicherung des Wasser und Lösungmittel enthaltenden Stroms in Schritt b) (ii) an Wasser derart durchgeführt wird, dass dieser Strom kondensiert und dann in eine wässrige Phase (250) und eine organische Phase (240) getrennt wird, wobei die organische Phase (240) optional weiter getrocknet werden kann, und bei welchem die Rückführung des nach Abreicherung von Wasser erhaltenen Stroms in Schritt A und/oder in Schritt B derart durchgeführt wird, dass die organische Phase (240), gegebenenfalls nach weiterer Trocknung, in Schritt A und/oder in Schritt B rezykliert wird.

12. Verfahren gemäß Anspruch 11, bei welchem die wässrige Phase (250) als Bestandteil des in Schritt B.VII) eingesetzten Absorptionsmittels eingesetzt wird.

13. Verfahren gemäß einem der Ansprüche 3 bis 12, bei welchem die Einrichtungen (2200) und (2300), sowie optional zusätzlich die Einrichtungen (2400) und, sofern vorhanden, (2410) bei einem gegenüber Atmosphärendruck vermindertem Druck betrieben werden und die Abgasströme der hierfür erforderlichen Vakuumerzeugungsanlagen ohne Abkühlung oder nach Abkühlung auf eine Temperatur nicht tiefer als 1,0 °C, optional nach Durchlaufen einer Vorrichtung zur katalytischen Phosgenzersetzung (3010), dem Schritt a) zugeführt werden.

14. Verfahren gemäß einem der vorstehenden Ansprüche, bei welchem das Amin (2) ausgewählt ist aus der Gruppe bestehend aus Methylendiphenylendiamin, Polymethylenpolyphenylenpolyamin, einem Gemisch aus Methylendiphenylendiamin und Polymethylenpolyphenylenpolyamin, Toluylendiamin, Xylylendiamin, 1,5-Pentandiamin, Hexamethylendiamin, Isophorondiamin und Naphthyldiamin.

15. Anlage (10000) zur Herstellung eines Isocyanats (1) durch Phosgenierung des korrespondierenden Amins (2), umfassend die Anlagenteile
0) optional, eine Vorrichtung (4000) zur Erzeugung von Phosgen (3) durch Umsetzung von Kohlenstoffmonoxid (300) mit Chlor (310), wobei ein Kohlenstoffmonoxid und Phosgen enthaltender Abgasstrom (320) anfällt;
I) eine Reaktionsstrecke (1000) umfassend einen Reaktionsraum (1200) zur Durchführung der Phosgenierung, dem optional eine Abscheideeinrichtung (1210) nachgeschaltet ist, wobei der Reaktionsraum oder die Abscheideeinrichtung mit Abfuhrleitungen für einen flüssigen Strom (60) und einen gasförmigen Strom (70) versehen sind;
II) eine Destillationsvorrichtung (2100) zur Auftrennung des flüssigen Stroms (60) in einen flüssigen Strom (80) und einen gasförmigen Strom (90);
III) optional, eine Destillationsvorrichtung (2200) zur Trennung des flüssigen Stroms (80) in einen gasförmigen Strom (110) und einen flüssigen Strom (100);
IV) optional, eine Destillationsvorrichtung (2300) zur Trennung des gasförmigen Stroms (110), optional nach dessen Verflüssigung in einem Kondensator (2310), in einen flüssigen Strom (120) und einen gasförmigen Strom (130);
V) optional, eine Destillationsvorrichtung (2400) zur Gewinnung eines flüssigen Isocyanat-Stroms (140) aus dem flüssigen Strom (100), wobei ein Nebenkomponenten und gegebenenfalls Lösungsmittel enthaltender gasförmiger Strom (150) anfällt, optional umfassend eine vorgeschaltete Einrichtung zur Polymerabtrennung (2410) zur Abtrennung von polymeren Isocyanatfraktionen (141);
VI) eine Vorrichtung zur Absorption (2500) der gasförmigen Ströme (70) und (90) und, sofern vorhanden, des gasförmigen Stroms (130), gegebenenfalls nach Durchlaufen von der Vorrichtung zur Absorption (2500) vorgeschalteten Einrichtungen (2510) zur Kondensation, in Lösungsmittel (4) unter Erhalt eines flüssigen Stroms (160) und eines gasförmigen Stroms (170);
VII) eine Vorrichtung zur Absorption (2600) des gasförmigen Stroms (170) in Wasser oder Salzsäure, optional umfassend einen Brüdenkondensator (2610), unter Erhalt eines Salzsäure und gegebenenfalls Lösungsmittel enthaltenden Stroms (190) und eines gasförmigen, Lösungsmittel und gegebenenfalls gasförmige Nebenkomponenten enthaltenden Stroms (200);
VIII) eine Vorrichtung zur Aufarbeitung von Abgasströmen (3000), umfassend
VIII-1) eine Vorrichtung zur katalytischen Phosgenzersetzung (3010) zur Zersetzung von in Strom (200) enthaltenen Phosgens und optional auch zur Zersetzung von in Strom (320) enthaltenen Phosgens unter Gewinnung eines gasförmigen, Lösungsmittel und gegebenenfalls gasförmige Nebenkomponenten enthaltenden Stroms (210);
VIII-2) eine Adsorptionsvorrichtung (3020) zur Adsorption von Lösungsmittel aus dem Strom (210), wobei die Adsorptionsvorrichtung (3020) mindestens zwei parallel geschaltete Adsorptionseinrichtungen (3021, 3022) umfasst, die so ausgestaltet sind, dass diese wechselweise
(i) mit dem Strom (210) beaufschlagt und
(ii) mit Wasserdampf regeneriert werden
können;
IX) eine Vorrichtung zur Abgasverbrennung (6000) zur Verbrennung des in VIII-2) (i) erhaltenen Gasstroms (220);
X) Einrichtungen zur Rezyklierung von in Vefahrensstufe VIII-2) (ii) desorbierten Lösungsmittels in die Reaktionsstrecke (1000) und/oder in die Vorrichtung zur Absorption (2500).

## Claims

1. Process for preparing an isocyanate (1), comprising the steps of:
A. reacting the amine (2) that corresponds to the isocyanate (1) with phosgene (3) using a solvent (4) to obtain a liquid stream comprising the isocyanate (1) and solvent (4) and also at least one solvent-containing offgas stream;
B. working up the liquid stream comprising isocyanate and solvent to isolate the isocyanate, wherein further solvent (4) may be used, likewise to obtain at least one solvent-containing offgas stream;
wherein
a) the at least one solvent-containing offgas stream from step A and/or from step B is guided into an adsorption apparatus for adsorption of solvent (3020) comprising at least two adsorption units (3021, 3022) connected in parallel that are alternately
(i) charged with the at least one offgas stream and
(ii) regenerated with steam,
wherein, during process stage a) (i), a solvent-depleted offgas stream (220) is obtained and, during process stage a) (ii), a stream comprising water and solvent (230) is obtained and
b)
(i) the solvent-depleted offgas stream (220) is sent to an offgas combustion (6000) and
(ii) the stream comprising water and solvent (230) is depleted of water and then recycled into step A and/or into step B.

2. Process according to Claim 1, in which step A comprises:
I) reacting the amine (2) with phosgene (3) and separating the process product obtained into the liquid stream (60) comprising the isocyanate and solvent, and a gaseous stream (70) comprising phosgene, hydrogen chloride and solvent;
and in which step B comprises:
II) depleting phosgene and hydrogen chloride from the liquid stream (60) from step I) by separating this liquid stream (60) into a liquid stream (80) comprising solvent and isocyanate, and a gaseous stream (90) comprising phosgene and hydrogen chloride in a distillation apparatus (2100);
wherein the gaseous streams (70) and (90), optionally after passing through further cleaning steps, are sent to step a) as a constituent, if appropriate as the sole constituent, of the at least one solvent-containing offgas stream.

3. Process according to Claim 2, in which step B.II) is followed by:
III) depleting solvent from the liquid stream (80) from step II) by separating this liquid stream (80) into a gaseous stream (110) comprising solvent, and a liquid stream (100) comprising isocyanate in a distillation apparatus (2200);
IV) depleting phosgene from the gaseous stream (110) from step III) by separating this gaseous stream (110), optionally after its liquefaction in a condenser (2310), into a liquid stream (120) comprising solvent and a gaseous stream (130) comprising phosgene in a distillation apparatus (2300);
wherein the gaseous stream (130), optionally after passing through further cleaning steps, is sent to step a) as a constituent of the at least one solvent-containing offgas stream.

4. Process according to Claim 3, in which step B.IV) is followed by:
V) obtaining a liquid isocyanate stream (140) from the liquid stream (100), resulting in a gaseous stream (150) comprising secondary components and optionally solvent, in a distillation apparatus (2400), optionally comprising the removal of polymeric isocyanate fractions in an upstream unit for polymer removal (2410) as stream (141).

5. Process according to any of Claims 2 to 4, in which step B additionally comprises:
VI) cleaning the gaseous streams (70) and (90) and, if present, the gaseous stream (130), optionally after condensation, by absorption in solvent (4) to obtain a liquid stream (160) comprising solvent and phosgene, and a gaseous stream (170) comprising hydrogen chloride and solvent in an absorption apparatus (2500).

6. Process according to Claim 5, in which step B.VI) is followed by:
VII) cleaning the gaseous stream (170) in water or hydrochloric acid as absorbent (180) in a further absorption apparatus (2600) to obtain a hydrochloric acid-containing stream (190) and, optionally after passing through a vapor condenser (2610), a gaseous stream (200) comprising solvent and optionally gaseous secondary components.

7. Process according to Claim 6, in which step B.VII) includes passage through the vapor condenser (2610) and this step additionally comprises:
separating the liquid stream (191) obtained in the vapor condenser (2610) into an aqueous phase (192) and an organic phase (193);
recycling the aqueous phase (192) as a constituent of the absorbent (180) into the absorption apparatus (2600) and/or
recycling the organic phase (193), optionally after drying, into at least one of steps A.I), B.III), B.IV and B.VI).

8. Process according to either of Claims 6 and 7, in which step B.VII) is followed by:
VIII) cleaning the gaseous stream (200) comprising solvent and optionally gaseous secondary components in an apparatus for workup of offgas streams (3000), wherein VII
I-1) the gaseous stream (200) that originates from the absorption apparatus (2600), optionally after passing through the vapor condenser (2610), is guided into an apparatus for phosgene decomposition (3010) in which phosgene is decomposed catalytically using water (260) to obtain a gaseous stream (210) comprising solvent and optionally gaseous secondary components and a liquid stream (270) comprising hydrochloric acid, wherein the gaseous stream (210) is guided into the adsorption apparatus (3020) for adsorption of solvent in step a).

9. Process according to any of the preceding claims, especially according to Claim 8, in which the phosgene (3) for the performance of step A is prepared by reacting carbon monoxide (300) with chlorine (310), giving an offgas stream (320) comprising carbon monoxide and phosgene which, optionally after passing through further cleaning steps, is fed into the at least one solvent-containing offgas stream to be sent to the offgas combustion.

10. Process according to Claim 9, in which the offgas stream (320) comprising carbon monoxide and phosgene from the reaction of carbon monoxide (300) with chlorine (310) is cleaned in solvent (4) having a temperature in the range from 0.0°C to -20.0°C in an absorption column (4010) to give a phosgene-depleted offgas stream (330) which, prior to performance of step VIII.1), is combined with the gaseous stream (200), giving a solvent-and phosgene-containing stream (340) in the absorption column (4010) which is sent to the absorption apparatus (2500).

11. Process according to any of the preceding claims, in which the depletion of water from the stream comprising water and solvent in step b) (ii) is conducted in such a way that this stream is condensed and then separated into an aqueous phase (250) and an organic phase (240), wherein the organic phase (240) may optionally be dried further, and in which the recycling of the stream obtained after depletion of water into step A and/or into step B is conducted in such a way that the organic phase (240), optionally after further drying, is recycled into step A and/or into step B.

12. Process according to Claim 11, in which the aqueous phase (250) is used as a constituent of the absorbent used in step B.VII).

13. Process according to any of Claims 3 to 12, in which the units (2200) and (2300), and optionally additionally the units (2400) and, if present, (2410) are operated at a reduced pressure relative to atmospheric pressure and the offgas streams from the vacuum generation plants that are required for this purpose are sent to step a) without cooling or after cooling to a temperature not lower than 1.0°C, optionally after passing through an apparatus for catalytic phosgene decomposition (3010).

14. Process according to any of the preceding claims, in which the amine (2) is selected from the group consisting of methylene diphenylene diamine, polymethylene polyphenylene polyamine, a mixture of methylene diphenylene diamine and polymethylene polyphenylene polyamine, tolylenediamine, xylylenediamine, pentane-1,5-diamine, hexamethylenediamine, isophoronediamine and naphthyldiamine.

15. Plant (10 000) for preparation of an isocyanate (1) by phosgenation of the corresponding amine (2), comprising the following plant components:
0) optionally an apparatus (4000) for production of phosgene (3) by reaction of carbon monoxide (300) with chlorine (310), giving an offgas stream (320) comprising carbon monoxide and phosgene;
I) a reaction zone (1000) comprising a reaction space (1200) for performance of the phosgenation, with a separation unit (1210) optionally connected downstream thereof, where the reaction space or the separation unit are provided with outlet conduits for a liquid stream (60) and a gaseous stream (70);
II) a distillation apparatus (2100) for separating the liquid stream (60) into a liquid stream (80) and a gaseous stream (90);
III) optionally a distillation apparatus (2200) for separating the liquid stream (80) into a gaseous stream (110) and a liquid stream (100) ;
IV) optionally a distillation apparatus (2300) for separating the gaseous stream (110), optionally after its liquefaction in a condenser (2310), into a liquid stream (120) and a gaseous stream (130);
V) optionally a distillation apparatus (2400) for obtaining a liquid isocyanate stream (140) from the liquid stream (100), resulting in a gaseous stream (150) comprising secondary components and optionally solvent, optionally comprising an upstream unit for polymer removal (2410) for removal of polymeric isocyanate fractions (141);
VI) an apparatus for absorption (2500) of the gaseous streams (70) and (90) and, if present, of the gaseous stream (130), optionally after passing through units (2510) for condensation connected upstream of the apparatus for absorption (2500), in solvent (4) to obtain a liquid stream (160) and a gaseous stream (170);
VII) an apparatus for absorption (2600) of the gaseous stream (170) in water or hydrochloric acid, optionally comprising a vapor condenser (2610), to obtain a stream (190) comprising hydrochloric acid and optionally solvent and a gaseous stream (200) comprising solvent and optionally gaseous secondary components;
VIII) an apparatus for workup of offgas streams (3000), comprising
VIII-1) an apparatus for catalytic phosgene decomposition (3010) for the decomposition of phosgene present in stream (200) and optionally also for the decomposition of phosgene present in stream (320) to obtain a gaseous stream (210) comprising solvent and optionally gaseous secondary components;
VIII-2) an adsorption apparatus (3020) for adsorption of solvent from stream (210), wherein the adsorption apparatus (3020) comprises at least two adsorption units (3021, 3022) connected in parallel, which are configured such that they can alternately be
(i) charged with stream (210) and
(ii) regenerated with steam;
IX) an apparatus for offgas combustion (6000) for combustion of the gas stream (220) obtained in VIII-2) (i);
X) units for recycling of solvent desorbed in process stage VIII-2) (ii) into the reaction zone (1000) and/or into the apparatus for absorption (2500).

## Revendications

1. Procédé pour la préparation d'un isocyanate (1), comprenant les étapes de :
A. transformation de l'amine (2) correspondant à l'isocyanate (1) avec du phosgène (3) avec utilisation d'un solvant (4), un flux liquide contenant l'isocyanate (1) et du solvant (4) ainsi qu'au moins un flux de gaz d'échappement contenant du solvant étant obtenus ;
B. traitement du flux liquide contenant l'isocyanate et du solvant pour l'isolement de l'isocyanate, du solvant (4) supplémentaire pouvant être utilisé, au moins un flux de gaz d'échappement étant également obtenu ;
a) l'au moins un flux de gaz d'échappement contenant du solvant de l'étape A et/ou de l'étape B étant conduit dans un dispositif d'adsorption pour l'adsorption de solvant (3020), qui comprend au moins deux dispositifs d'adsorption (3021, 3022) disposés en parallèle, qui sont en alternance
(i) soumis à l'au moins un flux de gaz d'échappement et
(ii) régénérés avec de la vapeur d'eau,
dans lequel pendant l'étape de procédé a) (i) un flux de gaz d'échappement (220) appauvri en solvant est obtenu et pendant l'étape de procédé a) (ii) un flux contenant de l'eau et du solvant (230) est obtenu et
b)
(i) le flux de gaz d'échappement (220) appauvri en solvant est alimenté à une combustion de gaz d'échappement (6000) et
(ii) le flux contenant de l'eau et du solvant (230) est appauvri en eau et ensuite reconduit dans l'étape A et/ou dans l'étape B.

2. Procédé selon la revendication 1, dans lequel l'étape A comprend :
I) la transformation de l'amine (2) avec du phosgène (3) et la séparation du produit de procédé obtenu en le flux liquide (60) contenant l'isocyanate et du solvant et un flux gazeux (70) contenant du phosgène, du chlorure d'hydrogène et du solvant ;
et dans lequel l'étape B comprend :
II) l'appauvrissement en phosgène et en chlorure d'hydrogène du flux liquide (60) de l'étape I) par séparation de ce flux liquide (60) en un flux liquide (80) contenant du solvant et l'isocyanate et un flux gazeux (90) contenant du phosgène et du chlorure d'hydrogène dans un dispositif de distillation (2100) ;
les flux gazeux (70) et (90), éventuellement après la soumission à des étapes de purification supplémentaires, étant alimentés à l'étape a) en tant qu'ingrédient, éventuellement en tant qu'ingrédient unique, de l'au moins un flux de gaz d'échappement contenant du solvant.

3. Procédé selon la revendication 2, dans lequel sont associés à l'étape B.II) :
III) un appauvrissement en solvant du flux liquide (80) de l'étape II) par séparation de ce flux liquide (80) en un flux gazeux (110) contenant du solvant et un flux liquide (100) contenant l'isocyanate dans un dispositif de distillation (2200) ;
IV) un appauvrissement en phosgène du flux gazeux (110) de l'étape III) par séparation de ce flux gazeux (110), éventuellement après sa liquéfaction dans un condensateur (2310), en un flux liquide (120) contenant du solvant et un flux gazeux (130) contenant du phosgène dans un dispositif de distillation (2300) ;
le flux gazeux (130), éventuellement après la soumission à des étapes de purification supplémentaires, étant alimenté à l'étape a) en tant qu'ingrédient de l'au moins un flux de gaz d'échappement contenant du solvant.

4. Procédé selon la revendication 3, dans lequel est associée à l'étape B.IV) :
V) l'obtention d'un flux liquide (140) d'isocyanate du flux liquide (100), un flux gazeux (150) contenant des composants secondaires et éventuellement du solvant étant produit, dans un dispositif de distillation (2400), éventuellement comprenant la séparation de fractions d'isocyanate polymériques dans un dispositif disposé en amont pour la séparation de polymère (2410) en tant que flux (141).

5. Procédé selon l'une quelconque des revendications 2 à 4, dans lequel l'étape B comprend de plus :
VI) la purification des flux gazeux (70) et (90) et, s'il est présent, du flux gazeux (130), éventuellement après condensation, par absorption dans du solvant (4) avec obtention d'un flux liquide (160) contenant du solvant et du phosgène et d'un flux gazeux (170) contenant du chlorure d'hydrogène et du solvant dans un dispositif d'absorption (2500) .

6. Procédé selon la revendication 5, dans lequel est associée à l'étape B.VI) :
VII) la purification du flux gazeux (170) dans de l'eau ou de l'acide chlorhydrique en tant qu'agent d'absorption (180) dans un dispositif d'absorption supplémentaire (2600), un flux (190) contenant de l'acide chlorhydrique et, éventuellement après la soumission à un condensateur de vapeurs (2610), un flux gazeux (200) contenant du solvant et éventuellement des composants secondaires gazeux étant obtenus.

7. Procédé selon la revendication 6, dans lequel dans l'étape B.VII), le condensateur de vapeurs (2610) est traversé et cette étape comprend de plus :
la séparation du flux liquide (191) obtenu dans le condensateur de vapeurs (2610) en une phase aqueuse (192) et une phase organique (193) ;
le recyclage de la phase aqueuse (192) en tant qu'ingrédient de l'agent d'absorption (180) dans le dispositif d'absorption (2600) et/ou le recyclage de la phase organique (193),
éventuellement après séchage, dans au moins l'une des étapes A.I), B.III), B.IV) et B.VI).

8. Procédé selon l'une quelconque des revendications 6 et 7, dans lequel est associée à l'étape B.VII) :
VIII) la purification du flux gazeux (200) contenant du solvant et éventuellement des composants secondaires gazeux dans un dispositif pour le traitement de flux de gaz d'échappement (3000),
VIII-1) le flux gazeux (200) provenant du dispositif d'absorption (2600), éventuellement après soumission au condensateur de vapeurs (2610), étant conduit dans un dispositif pour la décomposition de phosgène (3010), dans lequel le phosgène est décomposé de manière catalytique avec utilisation d'eau (260), un flux gazeux (210) contenant du solvant et éventuellement des composants secondaires gazeux et un flux liquide (270) contenant de l'acide chlorhydrique étant obtenus, le flux gazeux (210) étant conduit dans le dispositif d'adsorption (3020) pour l'adsorption de solvant dans l'étape a).

9. Procédé selon l'une quelconque des revendications précédentes, en particulier selon la revendication 8, dans lequel
le phosgène (3) pour la réalisation de l'étape A est préparé par transformation de monoxyde de carbone (300) avec du chlore (310), un flux de gaz d'échappement (320) contenant du monoxyde de carbone et du phosgène étant produit, qui, éventuellement après soumission à des étapes de purification supplémentaires, est alimenté à l'au moins un flux de gaz d'échappement contenant du solvant alimentant la combustion de gaz d'échappement.

10. Procédé selon la revendication 9, dans lequel le flux de gaz d'échappement (320) contenant du monoxyde de carbone et du phosgène provenant de la transformation de monoxyde de carbone (300) avec du chlore (310) dans du solvant (4) étant purifié à une température dans la plage de 0,0 °C à -20,0 °C dans une colonne d'absorption (4010) pour donner un flux de gaz d'échappement (330) appauvri en phosgène, qui est réuni avec le flux gazeux (200) avant la mise en œuvre de l'étape VIII.1), un flux (340) contenant du solvant et du phosgène étant produit dans la colonne d'absorption (4010), qui est alimenté au dispositif d'absorption (2500) .

11. Procédé selon l'une quelconque des revendications précédentes, dans lequel l'appauvrissement en eau du flux contenant de l'eau et du solvant dans l'étape b)(ii) est mis en œuvre de telle façon que ce flux est condensé et ensuite séparé en une phase aqueuse (250) et une phase organique (240), la phase organique (240) pouvant éventuellement être davantage séchée, et dans lequel le renvoi du flux obtenu après l'appauvrissement en eau dans l'étape A et/ou l'étape B est mis en œuvre de telle façon que la phase organique (240), éventuellement après un séchage supplémentaire, est recyclée dans l'étape A et/ou l'étape B.

12. Procédé selon la revendication 11, dans lequel la phase aqueuse (250) est utilisée comme ingrédient de l'agent d'absorption utilisé dans l'étape B.VII).

13. Procédé selon l'une quelconque des revendications 3 à 12, dans lequel les dispositifs (2200) et (2300), ainsi qu'éventuellement de plus les dispositifs (2400) et, s'il est présent, (2410) fonctionnent à une pression réduite par rapport à la pression atmosphérique et les flux de gaz d'échappement des installations de production de vide nécessaires pour cela sont alimentés à l'étape a) sans refroidissement ou après refroidissement à une température pas plus basse que 1,0 °C, éventuellement après soumission à un dispositif pour la décomposition catalytique de phosgène (3010).

14. Procédé selon l'une quelconque des revendications précédentes, dans lequel l'amine (2) est choisie dans le groupe constitué par la méthylènediphénylènediamine, une polyméthylènepolyphénylènepolyamine, un mélange de méthylènediphénylènediamine et de polyméthylènepolyphénylènepolyamine, la toluylènediamine, la xylylènediamine, la 1,5-pentanediamine, l'hexamethylènediamine, l'isophoronediamine et la naphtyldiamine.

15. Installation (10000) pour la préparation d'un isocyanate (1) par phosgénation de l'amine correspondante (2), comprenant les parties d'installation
0) éventuellement, un dispositif (4000) pour la production de phosgène (3) par transformation de monoxyde de carbone (300) avec du chlore (310), un flux de gaz d'échappement (320) contenant du monoxyde de carbone et du phosgène étant produit;
I) une section de réaction (1000) comprenant un espace de réaction (1200) pour la mise en œuvre de la phosgénation, en aval de laquelle éventuellement un dispositif de séparation (1210) est disposé, l'espace de réaction ou le dispositif de séparation étant pourvus de conduites d'évacuation pour un flux liquide (60) et un flux gazeux (70) ;
II) un dispositif de distillation (2100) pour la séparation du flux gazeux (60) en un flux liquide (80) et un flux gazeux (90) ;
III) éventuellement, un dispositif de distillation (2200) pour la séparation du flux liquide (80) en un flux gazeux (110) et un flux liquide (100) ;
IV) éventuellement, un dispositif de distillation (2300) pour la séparation du flux gazeux (110), éventuellement après sa liquéfaction dans un condensateur (2310), en un flux liquide (120) et un flux gazeux (130) ;
V) éventuellement, un dispositif de distillation (2400) pour l'obtention d'un flux liquide d'isocyanate (140) à partir du flux liquide (100), un flux gazeux (150) contenant des composants secondaires et éventuellement du solvant étant produit, éventuellement comprenant un dispositif disposé en amont pour la séparation de polymère (2410) pour la séparation de fractions d'isocyanate polymériques (141) ;
VI) un dispositif pour l'absorption (2500) des flux gazeux (70) et (90) et, s'il est présent, du flux gazeux (130), éventuellement après soumission à des dispositifs (2510) pour la condensation disposés en amont du dispositif pour l'absorption (2500), dans du solvant (4) avec obtention d'un flux liquide (160) et d'un flux gazeux (170) ;
VII) un dispositif pour l'absorption (2600) du flux gazeux (170) dans de l'eau ou de l'acide chlorhydrique, éventuellement comprenant un condensateur de vapeurs (2610), avec obtention d'un flux (190) contenant de l'acide chlorhydrique et éventuellement du solvant et d'un flux gazeux (200) contenant du solvant et éventuellement des composants secondaires gazeux ;
VIII) un dispositif pour le traitement de flux de gaz d'échappement (3000), comprenant VII
I-1) un dispositif pour la décomposition catalytique de phosgène (3010) pour la décomposition de phosgène contenu dans le flux (200) et éventuellement également pour la décomposition de phosgène contenu dans le flux (320) avec obtention d'un flux gazeux (210) contenant du solvant et éventuellement des composants secondaires gazeux ;
VIII-2) un dispositif d'adsorption (3020) pour l'adsorption de solvant du flux (210), le dispositif d'adsorption (3020) comprenant au moins deux dispositifs d'adsorption (3021, 3022) disposés en parallèle, qui sont conçus de telle manière que ceux-ci peuvent tour à tour
(i) être soumis au flux (210) et
(ii) être régénérés avec de la vapeur d'eau ;
IX) un dispositif pour la combustion de gaz d'échappement (6000) pour la combustion du flux de gaz (220) obtenu en VIII-2)(i) ;
X) des dispositifs pour le recyclage de solvant désorbé dans l'étape de procédé VIII-2)(ii) dans la section de réaction (1000) et/ou dans le dispositif pour l'absorption (2500).
